# EUROPEAN PATENT SPECIFICATION

(11) **EP 3 554 461 B1**
(45) Date of publication and mention of the grant of the patent: **24.03.2021**
(21) Application number: 17821551.3
(22) Date of filing: 15.12.2017
(51) Int. Cl.: A61K 8/31, A61Q 19/08, A61K 8/87, A61K 8/92, A61K 8/06

(54) **COSMETIC COMPOSITION COMPRISING SOLID FATTY SUBSTANCES AND A GELLING POLYMER**
KOSMETISCHE ZUSAMMENSETZUNG MIT FESTEN FETTSUBSTANZEN UND EINEM GELIERENDEN POLYMER
COMPOSITION COSMÉTIQUE COMPRENANT DES CORPS GRAS SOLIDES ET UN POLYMÈRE GÉLIFIANT

(30) Priority: 16.12.2016 FR 1662627
(43) Date of publication of application: 23.10.2019
(73) Proprietor: L'OREAL, 75008 Paris (FR)
(72) Inventor: GUIRAMAND, Carole, 94152 Chevilly la Rue (FR); BOILEAU, Nathalie, 94152 Chevilly la Rue (FR); HENAULT-MEZAIZE, Léonora, 94152 Chevilly la Rue (FR); CHABRILLANGEAS, Mathieu, 94152 Chevilly la Rue (FR); LORANT, Raluca, 94152 Chevilly la Rue (FR)
(74) Representative: L'Oreal
(86) International application number: PCT/EP2017/083167
(87) International publication number: WO 2018/109216

(56) References cited:
- WO-A2-2011/139433
- FR-A1- 2 834 208
- FR-A1- 2 981 567
- FR-A1- 2 983 715

## Description

The invention relates to a composition in the form of a fat phase-in-water emulsion comprising a mixture of solid fatty substances and of thickening polymers. The invention also relates to the use of such a composition for treating the skin.

In the course of the process of ageing, impairment of the structure and functions of the skin appears, giving rise to slackening of the skin, which is manifested especially by the loss of elasticity of the skin and by the loss of volume of the face, and also by the appearance of wrinkles and fine lines. A person skilled in the art knows that such slackening can be corrected immediately by applying a tensioning agent to the skin.

At the present time, the use of numerous tensioning polymers for treating wrinkles is known to those skilled in the art. Acrylic-grafted silicone polymers or interpenetrating polymer networks are thought of in particular for smoothing out wrinkles via a tensioning effect, described especially in EP 1 038 519 and FR 2 843 025.

Unfortunately, compositions containing such tensioning agents, although having a very satisfactory immediate tensioning effect, have the drawback of presenting a certain level of discomfort to the user on account of the skin tautness generated by the tensioning polymer. Furthermore, the tensioning polymer film does not always have good persistence on account of its fragmentation over time giving rise to cracking and fluffing, and also a loss of efficacy over time of the tensioning effect.

Consequently, there is a need for cosmetic compositions that can efficiently treat skin slackening in a manner that is comfortable to the user.

The Applicant has discovered that a composition comprising a particular mixture of solid fatty substances and particular thickening polymers as defined below makes it possible, after application to the skin, in particular to facial skin, to provide a surface covering for the skin, which maintains the skin at the surface by providing a sensation of containment. The skin is less slack, tighter and firmer. The covering effect also gives rise to tightening of the skin pores. The skin is enveloped with a supple, comfortable (absence of tautness) and invisible covering film. The covering effect of the applied composition contributes towards reducing the appearance of the fine lines of the skin, in particular the fine lines close to the eyes. As soon as the cream is applied to the skin, the user feels the surface covering effect affording a sensation of restructuring and redensifying of the skin tissues. The covering effect is perceived on each application of the composition to the skin: it is felt by the user even after several weeks of use.

The composition applied to the skin also gives the skin a matt effect, and also an effect of concealing and attenuating skin defects such as fine lines and pores.

In addition, the composition according to the invention has good stability, especially after storage for one month, or even two months, at room temperature (25°C) and at 45°C. The composition remains homogeneous and does not show any phase separation or any release of oil at the surface of the composition. The hardness of the composition as described below varies little.

Furthermore, during application of the composition to the skin, the composition is easy to spread on account of the fondant texture of the composition, thus allowing uniform distribution of the product on the surface of the skin: it penetrates easily into the skin. After application, the applied product is not tacky or greasy and has a soft, pleasant feel, without any sensation of heaviness.

More specifically, the present invention relates to a composition in the form of a fatty phase-in-water emulsion comprising:
(a) a first solid fatty substance with a hardness, at 25°C, ranging from 0.2 to 2 MPa;
(b) a second solid fatty substance with a hardness, at 25°C, ranging from 5 to 15 MPa;
(c) an associative aqueous gelling polymer.

The present invention also relates to a process for preparing said composition.

The present invention similarly relates to a process for the treatment, especially the cosmetic treatment, of the skin, comprising the application of the composition described previously to the skin. The skin treatment process is in particular a process for treating slackening of the skin and/or for reducing the appearance of the fine lines of the skin.

The process is in particular performed on the skin of the face (cheeks, forehead, chin, around the eyes) and the neck.

### Fatty phase

The composition according to the invention comprises a fatty phase which comprises at least two different solid fatty substances as defined below.

### Solid fatty substances:

The composition according to the invention comprises at least one first solid fatty substance with a hardness, at 25°C, ranging from 0.2 to 2 MPa and at least one second solid fatty substance with a hardness ranging from 5 to 15 MPa.

The hardness of the solid fatty substance is measured according to the following protocol:
An amount of about 15 g of solid fatty substance is heated to 85°C, poured into an aluminium capsule 75 mm in diameter and 15 mm deep and then left to cool for 24 hours at room temperature (25°C). The capsule is filled to 75%.

The hardness the solid fatty substance is measured with a TA/TX2i Plus texturometer (Swantech) equipped with a cylindrical spindle chosen according to the texture of the solid fatty substance:
- a cylindrical steel spindle 2 mm in diameter for hard solid fatty substances;
- a cylindrical steel spindle 12 mm in diameter for soft solid fatty substances.

The measurement comprises three steps: a first step after automatic detection of the surface of the sample, where the spindle moves at a measuring speed of 0.1 mm/s, and penetrates into the solid fatty substance to a depth of 0.3 mm, the software notes the maximum compression force value reached; a second "relaxation" step where the spindle remains at this position for one second and the force is noted after 1 second of relaxation; finally, a third "withdrawal" step in which the spindle returns to its initial position at a speed of 1 mm/s, and the spindle withdrawal energy (negative force) is noted.
The hardness value corresponds to the maximum compression force measured in newtons divided by the area of the texturometer cylinder expressed in mm² in contact with the solid fatty substance. The hardness value obtained is expressed in megapascals or MPa.
Five measurements are taken on each sample, and the mean of the five measurements is then calculated.

Advantageously, the first solid fatty substance has a melting point below or equal to 45°C (especially ranging from 28 to 45°C), preferably ranging from 28 to 40°C.
Advantageously, the second solid fatty substance has a melting point above 45°C, preferably above 45°C and below or equal to 90°C.

The solid fatty substances may be chosen from pasty fatty substances and waxes.

For the purposes of the present invention, the term "pasty" refers to a water-immiscible compound with a reversible solid/liquid change of state, having anisotropic crystalline organization in the solid state, and comprising, at a temperature of 23°C, a liquid fraction and a solid fraction. The pasty fatty substance may be chosen from synthetic pasty compounds and fatty substances of plant origin. The pasty fatty substance may be a hydrocarbon-based or silicone substance.

For the purposes of the present invention, the term "wax" means a lipophilic compound that is solid at room temperature (25°C), with a reversible solid/liquid change of state. For the purposes of the invention, the melting point corresponds to the temperature of the most endothermic peak observed on thermal analysis (DSC) as described in standard ISO 11357-3; 1999. The melting point of a solid fatty substance may be measured using a differential scanning calorimeter (DSC), for example the calorimeter sold under the name DSC Q100 by the company TA Instruments, with the TA Universal Analysis software.

The measurement protocol is as follows:
A sample of about 5 mg of solid fatty substance is placed in a "hermetic aluminium capsule" crucible.

When the solid fatty substance is soft (pasty fatty substance), the sample is subjected to a first temperature rise going from 20°C to 80°C, at a heating rate of 2°C/minute up to 80°C, it is left at the 80°C isotherm for 20 minutes and is then cooled from 80°C to -80°C at a cooling rate of 2°C/minute, and is finally subjected to a second temperature rise going from -80°C to 20°C at a heating rate of 2°C/minute.

When the solid fatty substance is hard (wax), the sample is subjected to a first temperature rise going from 20°C to 120°C, at a heating rate of 2°C/minute up to 80°C, it is left at the 120°C isotherm for 20 minutes and is then cooled from 120°C to 0°C at a cooling rate of 2°C/minute, and is finally subjected to a second temperature rise going from 0°C to 20°C at a heating rate of 2°C/minute.

The melting point value of the solid fatty substance is the value at the top of the most endothermic peak observed in the melting curve, representing the variation of the difference in power absorbed as a function of the temperature.

The waxes that may be used in the composition according to the invention may be waxes of animal, plant, mineral or synthetic origin. The waxes may be hydrocarbon-based, silicone and/or fluoro waxes.

The first solid fatty substance may be chosen from:
- butters of plant origin, for instance mango butter, such as the product sold under the reference Lipex 203 by the company Aarhuskarlshamn or Trivent Mango Butter by the company Alzo, shea butter, in particular the product whose INCI name is *Butyrospermum parkii* Butter, such as the product sold under the reference Sheasoft® or Lipex 102 by the company Aarhuskarlshamn, cupuacu butter such as the product sold under the name Rain Forest RF3410 by the company Beraca Sabara, murumuru butter such as Rain Forest RF3710 from the company Beraca Sabara, cocoa butter such as the product sold under the name CT Cocoa Butter Deodorized by the company Dutch Cocoa BV, babassu butter such as the product sold under the name Cropure Babassu SS-(LK) by Croda, avocado butter, kokum butter, coconut butter, apricot kernel butter, *Shorea robusta* butter such as the product sold under the name Dub Shorea T by the company Stéarineries Dubois (INCI name: *Shorea robusta* Seed Butter), urucum butter; the mixture of mimosa, jojoba and sunflower plant waxes, for instance the mixture sold under the name Acticire by Gattefosse; and mixtures thereof;
- (totally or partially) hydrogenated plant oils, for instance hydrogenated soybean oil, hydrogenated coconut oil, hydrogenated rapeseed oil, hydrogenated jojoba oil, for instance the product sold by the company Desert Whale under the commercial reference Iso-Jojoba-50® (INCI name: *Simmondsia chinensis* (jojoba) Butter), hydrogenated olive oil, for instance the product sold under the reference Beurrolive or Inholive by the company Soliance, mixtures of hydrogenated plant oils such as the mixture of hydrogenated soybean, coconut, palm and rapeseed plant oils, for instance the mixture sold under the reference Akogel®;
- petroleum jelly,
- C₂-C₄ polyalkylene glycol pentaerythrityl ethers, especially containing from 3 to 10 oxyalkylene units, preferably from 3 to 10 oxyethylene or oxypropylene units, for instance polyethylene glycol pentaerythrityl ether comprising 5 oxyethylene units (5 OE) (CTFA name: PEG-5 Pentaerythrityl Ether), polypropylene glycol pentaerythrityl ether comprising 5 oxypropylene (5 OP) units (CTFA name: PPG-5 Pentaerythrityl Ether) and mixtures thereof, and more especially the mixture PEG-5 Pentaerythrityl Ether, PPG-5 Pentaerythrityl Ether and soybean glycine sterols, sold under the name Lanolide by the company Vevy, which is a mixture in which the constituents are in a 46/46/8 weight ratio: 46% PEG-5 pentaerythrityl ether, 46% PPG-5 pentaerythrityl ether and 8% soybean glycine sterols,
- block copolymers of ethylene oxide and/or propylene oxide and of an alkylene oxide containing from 6 to 40 carbon atoms, the copolymer having a weight-average molecular weight ranging from 5000 to 8000.
   Advantageously, the alkylene oxide of the copolymer may comprise from 6 to 30 carbon atoms, preferably from 8 to 20 carbon atoms, preferentially from 10 to 18 carbon atoms and more preferentially from 10 to 14 carbon atoms.
   The weight-average molecular weight of the copolymer ranges from 5000 to 8000, preferably from 5500 to 7000, preferentially from 5500 to 6500 and more preferentially from 5800 to 6200. The copolymer advantageously comprises from 35 to 55 ethylene oxide and/or propylene oxide units and from 15 to 30 alkylene oxide units containing from 6 to 40 carbon atoms.
   In particular, the copolymer is such that the ratio between the number of ethylene oxide and/or propylene oxide units and the number of alkylene oxide units containing from 6 to 40 carbon atoms may range from 1.5 to 2.5, preferably range from 1.8 to 2.3 and preferentially range from 1.9 to 2.1.

Such copolymers are especially described in FR-A-2 425 848. Use is preferably made of a block copolymer of polyoxyethylene/polydodecyl glycol such as the copolymers of dodecanediol (22 mol) and of polyethylene glycol (45 OE) sold under the brand name Elfacos ST9 by AkzoNobel (INCI name: PEG-45/dodecyl glycol copolymer).
- esters derived from the condensation of a linear or branched, preferably saturated, C6-C10 dicarboxylic acid and of an ester of diglycerol and of optionally hydroxylated, linear or branched, preferably saturated, C6-C20 monocarboxylic acids, in particular the diester obtained by condensation of adipic acid and of the ester of diglycerol and of a mixture of C6-C20 fatty acids such as stearic acid, capric acid, stearic acid, isostearic acid and 12-hydroxystearic acid, sold especially under the reference Softisan® 649 by the company Cremer Oleo (INCI name: Bis-diglyceryl polyacyladipate-2),
- vinyl ester homopolymers bearing C8-C30 alkyl groups, such as polyvinyl laurate (sold especially under the reference Mexomer PP by the company Chimex),
- copolymers of allyl stearate and of vinyl acetate, such as the product sold under the name Mexomer PQ by the company Chimex,
- arachidyl propionate such as the product sold under the brand name Waxenol 801 by Alzo,
- triglycerides of optionally hydrogenated (totally or partially) C₆-C₃₀, more particularly C₈-C₁₈ and preferably C₁₂-C₁₈, optionally monohydroxylated or polyhydroxylated, linear or branched, saturated or unsaturated fatty acids; for instance the saturated C₁₂-C₁₈ fatty acid glycerides sold under the name Softisan 100® by the company Cremer Oleo (INCl name: Hydrogenated cocoyl glycerides),
- pentaerythritol esters chosen from esters of pentaerythritol and of a C8-C22 fatty acid, esters of pentaerythritol and of a C4-C10 dicarboxylic acid, and mixtures thereof, such as the pentaerythrityl isostearate/caprate/caprylate/adipate mixture sold under the name Supermol L-LQ-(RB) by the company Croda;
- esters of a dimer of dilinoleyl alcohol and of dilinoleic acid in which the hydroxyl groups are esterified with a mixture of phytosterols, of behenyl alcohol and of isostearyl alcohol, for example the ester sold under the name Plandool G by the company Nippon Fine Chemical (INCI name: Bis-behenyl/isostearyl/phytosteryl dimer dilinoleyl dimer dilinoleate);
- esters of dilinoleic acid and of a mixture of phytosterols, isostearyl alcohol, cetyl alcohol, stearyl alcohol and behenyl alcohol, for example the ester sold under the name Plandool H or Plandool S by the company Nippon Fine Chemical (INCI name: Phytosteryl/isostearyl/cetyl/stearyl/behenyl dimer dilinoleate)
- esters of hydrogenated castor oil and of dilinoleic acid dimer, such as the products sold under the names Risocast-DA-L or Risocast-DA-H by the company Kokyu Alcohol Kogyo (INCI name: Hydrogenated castor oil dimer dilinoleate);
- esters of hydrogenated castor oil and of C16-C22 fatty acids, in particular of isostearic acid, for instance Salacos HCIS (V-L) sold by the company Nisshin Oil;
- waxes of plant origin, for instance laurel wax, such as the product sold under the name Laurel Wax from the company Linea Vegetal Mukuna; orange wax, such as the product sold under the name Orange Peel Wax by the company Koster Keunen;
- mineral waxes, for instance microcrystalline wax, such as the product sold under the name White Microcrystalline Wax SP-18 by the company Strahl & Pitsch;
- vinylpyrrolidone/eicosene copolymer, such as the product sold under the name Antaron V 220F or Ganex 220F by ISP;
and mixtures thereof.

According to a preferred embodiment, the first solid fatty substance is chosen from:
- butters of plant origin, and in particular mango butter such as the product sold under the name Trivent Mango Butter by the company Alzo, cocoa butter such as the product sold under the name CT Cocoa Butter Deodorized by the company Dutch Cocoa BV, the mixture of mimosa, jojoba and sunflower plant waxes, for instance the mixture sold under the name Acticire by Gattefosse;
- (totally or partially) hydrogenated plant oils, for instance hydrogenated soybean oil, hydrogenated coconut oil, hydrogenated rapeseed oil or hydrogenated jojoba oil;
   and mixtures thereof.

Preferentially, the first solid fatty substance is chosen from mango butter, cocoa butter; the mixture of mimosa, jojoba and sunflower plant waxes; hydrogenated jojoba oil; and mixtures thereof.

The first solid fatty substance may be present in the composition according to the invention in a content ranging from 0.5% to 15% by weight, preferably ranging from 1% to 10% by weight and better still from 2% to 6% by weight relative to the total weight of the composition.

The second solid fatty substance may be chosen from fatty substances with a hardness, at 25°C, ranging from 5 to 15 MPa, preferably ranging from 6 to 12 MPa.

The second solid fatty substance may be chosen from:
- esters of ethylene glycol and of a C12-C30 fatty acid, such as ethylene glycol monopalmitate, for instance the product sold under the name Lanol P Ecailles by the company SEPPIC;
- waxes of animal origin, for instance beeswax, such as the product sold under the name White Beeswax SP 453P by Strahl & Pitsch, or under the name Cyclochem 326 A by the company Evonik Goldschmidt or under the name Kester Wax K 82 H, Kester Wax K 82 P-VS or Kester Wax K 82 P by the company Koster Keunen; polyglycerolated beeswax, such as the polyglycerolated (3 mol) beeswax sold especially under the reference Cerabellina by the company Koster Keunen; silicone beeswaxes, such as beeswax esterified with a dimethicone copolyol, in particular a PEG-12 dimethicone, such as the product sold under the name Siliconyl Beeswax by the company Koster Keunen, beeswax esterified with dimethiconol, for instance the product sold under the reference Ultrabee WD Silicone by the company Lubrizol; insect wax, such as the product sold under the name Chinese Insect Wax by the company Kahl; shellac wax, for instance the product sold under the name Shellac Wax 7302 L by the company Kahl;
- C30-45 alkyl dimethicones, for instance the product sold under the name SF 1642 by the company Momentive Performance Materials;
- fatty alcohols comprising from 16 to 50 carbon atoms, preferably from 16 to 22 carbon atoms, and mixtures thereof, for instance cetyl alcohol, stearyl alcohol; behenyl alcohol; arachidyl alcohol (C20), and mixtures thereof;
- waxes of mineral origin, such as ozokerite, for instance the product sold under the reference Paracera ARFB by the company Paramelt or the product sold under the name Ozokerite 313 by the company Cires et Derives; microcrystalline wax, such as the products sold under the names Paracera M and Microwax HW by the company Paramelt; ceresin wax, for instance the product sold under the name White Ceresin Wax JH 520 by the company Hansotech; montan wax, for instance the product sold under the name Montan Wax SP 48 by the company Saint Gobain;
- waxes of plant origin such as candelilla wax, for instance the product sold under the reference Candelilla Wax SP 75 G by the company Strahl & Pitsch; carnauba wax such as the products sold under the names Cerauba T1, Cerauba T1 Bio and Cerauba T3 by the company Baerlocher; rice bran wax, such as the product sold under the name NC 1720 by the company Cera Rica Noda; sunflower wax, such as the product sold under the name Sunflower Wax by the company Koster Keunen; lemon wax, such as the product sold under the name Lemon Peel Wax (Wax# 288B) by the company Koster Keunen Holland BV; ouricury wax sold especially under the name Ouricury Wax by Strahl & Pitsch; macadamia wax, such as the product sold under the name Macadamia Wax by the company Provital;
- hydrogenated plant oils, for instance hydrogenated jojoba oil, such as the product sold under the name Jojoba Wax Flakes by Desert Whale; hydrogenated castor oil, for instance the product sold under the name Cutina HR Powder by Cognis or under the name Castor Wax MP 80 by the company Caschem; hydrogenated palm oil, for instance the product sold under the name GV 60 by the company SIO; hydrogenated olive oil, such as the product sold under the name Waxolive by the company Soliance;
- synthetic waxes, such as polyethylene wax, for instance the products sold under the name Performalene 400 Polyethylene, Performalene 500 L Polyethylene or Performalene 655 Polyethylene by New Phase Technologies; paraffin wax, such as the product sold under the name Sasol Wax 5603 by Sasol; polymethylene wax, such as the product sold under the name Cirebelle 303 by the company Cirebelle;
- esters of a C14-C30 fatty acid and of a plant oil, for instance hydrogenated castor oil isostearate, such as the product sold under the name Kama-MIS A by the company Ethox Chemicals; behenic ester of castor oil, such as the product sold under the name Phytowax Ric in 22 L 73 by the company Sophim; cetyl ester of castor oil, such as the product sold under the name Phytowax Ricin 16 L 64 by the company Sophim; stearic esters of hydrogenated olive oil, for instance Phytowax Olive 18 L 57 sold by the company Sophim; myristic esters of hydrogenated olive oil, for instance the product sold under the name Phytowax Olive 14 L 48 by the company Sophim;
- polymers, especially homopolymers, of C10 to C30 and preferably C14 to C22 alkyl (meth)acrylate; and more particularly poly(stearyl acrylate) or poly(behenyl acrylate) homopolymers, such as the products sold under the names Intelimer® IPA 13-1 NG Polymer and Intelimer® IPA 13-6 by the company Air Products and Chemicals (INCI name: Poly C10-30 alkyl acrylate).

Use is preferentially made of poly(stearyl acrylate) homopolymers;
- and mixtures thereof.

Preferably, the second solid fatty substance is chosen from waxes of animal origin and in particular white beeswax; C16-C50 fatty alcohols and in particular stearyl alcohol; mineral waxes and in particular ozokerite, microcrystalline wax; plant waxes such as candelilla wax, carnauba wax; hydrogenated jojoba oil; polyethylene wax; C10 to C30 alkyl (meth)acrylate homopolymers; and mixtures thereof.

In a particular embodiment of the invention, the second solid fatty substance is chosen from plant waxes such as candelilla wax or carnauba wax; mineral waxes and in particular ozokerite; C10 to C30 alkyl (meth)acrylate homopolymers (poly(stearyl acrylate) or poly(behenyl acrylate) homopolymers), and mixtures thereof.

According to one embodiment of the invention, the second solid fatty substance comprises a C10 to C30 alkyl (meth)acrylate homopolymer, in particular a poly(stearyl acrylate) or poly(behenyl acrylate) homopolymer, and preferentially a poly(stearyl acrylate) homopolymer; optionally combined with a plant wax or a mineral wax such as those described previously.

The second solid fatty substance may be present in the composition according to the invention in a content ranging from 0.5% to 15% by weight, preferably ranging from 1% to 10% by weight and better still from 2% to 8% by weight, relative to the total weight of the composition.

Advantageously, the first and second solid fatty substances may be present in the composition according to the invention in a total content ranging from 6% to 25% by weight, relative to the total weight of the composition, preferably ranging from 6% to 20% by weight, preferentially ranging from 8% to 16% by weight and more preferentially ranging from 10% to 14%.

According to one embodiment, the first and second solid fatty substances are present in the composition according to the invention in a mass ratio of first solid fatty substance to second solid fatty substance ranging from 0.4 to 0.8, preferably ranging from 0.45 to 0.75 and preferentially ranging from 0.5 to 0.7.

### Associative aqueous gelling polymer:

The composition according to the invention comprises at least one associative hydrophilic gelling polymer. For the purposes of the present invention, the term "associative polymer" means an amphiphilic polymer that is capable, in an aqueous medium, of reversibly combining with itself or with other molecules. It generally comprises, in its chemical structure, at least one hydrophilic region or group and at least one hydrophobic region or group.

The term "hydrophobic group" means a group or a polymer bearing a saturated or unsaturated and linear or branched hydrocarbon-based chain. When it denotes a hydrocarbon-based group, the hydrophobic group comprises at least 10 carbon atoms, preferably from 10 to 30 carbon atoms, in particular from 12 to 30 carbon atoms and preferentially from 18 to 30 carbon atoms. Preferentially, the hydrocarbon-based hydrophobic group originates from a monofunctional compound. By way of example, the hydrophobic group may be derived from a fatty alcohol, such as stearyl alcohol, dodecyl alcohol or decyl alcohol, or else from a polyalkylenated fatty alcohol, such as Steareth-100. It may also denote a hydrocarbon-based polymer, such as, for example, polybutadiene.

The term "hydrophilic gelling polymer" means a polymer that is capable of thickening an aqueous medium.

Advantageously, the associative hydrophilic gelling polymer is chosen from nonionic associative polyurethane polyethers, C12-C20 fatty alcohol ethers of nonionic hydroxyethylcellulose and anionic associative acrylic polymers.

Among the anionic associative acrylic polymers, mention may be made of:
(1) copolymers derived from the polymerization of:
   (i) (meth)acrylic acid,
   (ii) a monomer of formula (II) below:

      CH₂ = CR'CH₂OBₙR (II)

      in which R' denotes H or CH₃, B denotes the ethyleneoxy group (-CH₂-CH₂-O-), n is zero or denotes an integer ranging from 1 to 100 (especially from 5 to 15) and R denotes a hydrocarbon-based group chosen from alkyl, arylalkyl, aryl, alkylaryl and cycloalkyl groups comprising from 8 to 30 carbon atoms, preferably from 10 to 24 carbon atoms and even more particularly from 16 to 20 carbon atoms.

   A monomer of formula (II) that is more particularly preferred is a monomer in which R' denotes H, n is equal to 10 and R denotes a stearyl (C18) group.
   Such anionic associative polymers are described in patent EP-0 216 479.
   Among these anionic associative polymers, the ones that are particularly preferred are polymers formed from 20% to 60% by weight of (meth)acrylic acid, from 5% to 60% by weight of C₁-C₄ alkyl (meth)acrylate, from 2% to 50% by weight of monomer of formula (II), and from 0 to 1% by weight of a crosslinking agent which is a well-known copolymerizable unsaturated polyethylenic monomer, for instance diallyl phthalate, allyl (meth)acrylate, divinylbenzene, (poly)ethylene glycol dimethacrylate or methylenebisacrylamide.
   Among the latter polymers, preference is given most particularly to terpolymers of methacrylic acid, ethyl acrylate and polyoxyethylenated stearyl alcohol allyl ether containing 10 mol of ethylene oxide (INCI name: Steareth-10 allyl ether/acrylates copolymer), especially in 40/50/10 respective weight proportions, such as the product sold under the name Salcare SC 80 by the company Ciba.
(2) associative polymers comprising at least one hydrophilic unit of unsaturated ethylenic carboxylic acid type and at least one hydrophobic unit of (C₁₀-C₃₀)alkyl ester of unsaturated carboxylic acid type.
   Preferably, these polymers are chosen from copolymers of (i) a monomer of formula (III) below: 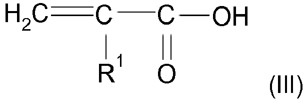 in which R¹ denotes H, CH₃ or C₂H₅, and of (ii) a monomer of formula (IV) below (monomer of (C₁₀-C₃₀)alkyl ester of unsaturated carboxylic acid type):

   H₂C=CR¹-COOR³ (IV)

   in which R¹ denotes H or CH₃ or C₂H₅ and preferably H or CH₃, R³ denotes a C₁₀-C₃₀ and preferably C₁₂-C₂₂ alkyl group.
   In this polymer, the monomer (III) constitutes the hydrophilic unit and the monomer (IV) constitutes the hydrophobic unit.
   (C₁₀-C₃₀)Alkyl esters of unsaturated carboxylic acids comprise, for example, lauryl (meth)acrylate, stearyl (meth)acrylate, decyl (meth)acrylate, isodecyl (meth)acrylate and dodecyl (meth)acrylate.
   Anionic polymers of this type are described and prepared, for example, according to patents US 3 915 921 and 4 509 949. Among the anionic associative polymers of this type that will be used more particularly are polymers formed from a monomer mixture comprising:
   (i) acrylic acid,
   (ii) an ester of formula (IV) described above in which R¹ denotes H or CH₃ and R³ denotes an alkyl group containing from 12 to 22 carbon atoms,
   (iii) and optionally a crosslinking agent, which is a well-known copolymerizable polyethylenic unsaturated monomer, such as diallyl phthalate, allyl (meth)acrylate, divinylbenzene, (poly)ethylene glycol dimethacrylate or methylenebisacrylamide.
   Among the anionic associative polymers of this type, use will be made more particularly of:
   those constituted of 95% to 60% by weight of acrylic acid, 4% to 40% by weight of a C₁₀-C₃₀ alkyl acrylate and 0 to 6% by weight of a crosslinking polymerizable monomer,
   or alternatively those constituted of 98% to 96% by weight of acrylic acid, 1% to 4% by weight of a C₁₀-C₃₀ alkyl acrylate and 0.1% to 0.6% by weight of a crosslinking polymerizable monomer, such as those described previously.

   Among the abovementioned polymers, the ones that are most particularly preferred are the products sold by the company Lubrizol under the trade names Pemulen TR1, Pemulen TR2, Carbopol 1382, Carbopol ETD 2020, Carbopol Ultrez 20 and Carbopol Ultrez 21 (INCI name: Acrylates /C10-30 alkyl acrylate crosspolymer), and even more preferentially Pemulen TR1 and Carbopol 1382.
(3) acrylic terpolymers comprising:
   (a) from 19.5% to 70% by weight of an α,β-monoethylenically unsaturated carboxylic acid containing from 3 to 5 carbon atoms,
   (b) from 20% to 80% by weight of C₁-C₄ alkyl (meth)acrylates,
   (c) 0.5% to 60% by weight of a nonionic urethane macromonomer of formula (IV) below: 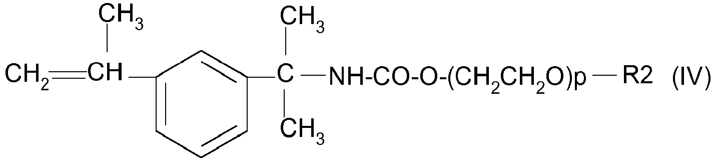 in which p ranges from 6 to 150 and R2 is chosen from linear alkyl radicals comprising from 18 to 26 and preferably from 20 to 24 carbon atoms. Preferably, the radical R2 is a behenyl radical.

   Such terpolymers are described especially in patent application EP-A-0 173 109.
   The α,β-monoethylenically unsaturated carboxylic acid (a) may be chosen from acrylic acid, methacrylic acid and crotonic acid. It is preferably (meth)acrylic acid. Preferentially, the monomer (a) is methacrylic acid.
   The terpolymer contains a monomer (b) chosen from C₁-C₄ alkyl (meth)acrylates such as methyl (meth)acrylate, ethyl (meth)acrylate or butyl (meth)acrylate. The monomer (b) is preferably chosen from methyl acrylate and ethyl acrylate.
   Such terpolymers are generally in the form of an aqueous dispersion.
   Use is preferentially made of a terpolymer of methacrylic acid/methyl acrylate/condensate of dimethyl meta-isopropylenyl benzyl isocyanate and of polyoxyethylenated (40 OE) behenyl alcohol (INCI name: Polyacrylate-3), such as the product sold in the form of an aqueous dispersion at 25% by weight, under the name Viscophobe DB 1000 by the company The Dow Chemical Company.
(4) copolymers of an α,β-monoethylenically unsaturated carboxylic acid and of an ester of an α,β-monoethylenically unsaturated carboxylic acid and of a polyoxyethylenated C12-C30 fatty alcohol, especially with 10 to 50 ethylene oxide units, and of an ester of an α,β-monoethylenically unsaturated carboxylic acid and of a C₁-C₄ alcohol.
   Examples of such copolymers that may be mentioned include:
   polymers of acrylic acid, of methyl acrylate and of 20 OE polyoxyethylenated stearyl methacrylate crosslinked with pentaerythrityl allyl ether or trimethylolpropane allyl ether (INCl name: Acrylates/steareth-20 methacrylate crosspolymer) sold under the name Aculyn 88 Polymer by the company The Dow Chemical Company,
   crosslinked polymers of acrylic acid, of methyl acrylate and of 25 OE polyoxyethylenated behenyl methacrylate (INCI name: Acrylates/beheneth-25 methacrylate copolymer), such as the product sold under the name Novethix L-10 Polymer by the company Lubrizol Advanced Materials, Inc.,
   polymers of acrylic acid, of methyl acrylate and of 25 OE polyoxyethylenated C12-C24 alkyl methacrylate (INCI name: Acrylates/palmeth-25 acrylate copolymer), such as the product sold under the name Synthalen W2000 L by the company 3V Group,
   polymers of methacrylic acid, of ethyl methacrylate, of polyethylene glycol C16-C22 alkyl ether methacrylate containing 25 ethylene glycol units, of the ether of 2-(6,6-dimethylbicyclo[3.1.1]hept-2-en-2-yl)ethyl methacrylate and of polypropylene glycol containing 5 propylene glycol units and of polyethylene glycol containing 25 ethylene glycol units (INCl name: Polyacrylate-33), such as the product sold under the name Rheomer® 33 by the company Rhodia Novecare.
      - the polyoxyethylenated (20 OE) terpolymer of acrylic acid/ethyl acrylate/stearyl methacrylate (INCI name: Acrylates/steareth-20 methacrylate copolymer) sold especially under the name Aculyn 22 by the company The Dow Chemical Company,
      - the polyoxyethylenated (25 OE) terpolymer of acrylic acid/ethyl acrylate/behenyl methacrylate (INCI name: Acrylates/beheneth-25 methacrylate copolymer) sold especially under the name Aculyn 28 Polymer by the company The Dow Chemical Company.
(5) copolymers of (meth)acrylic acid, of crosslinked C1-C4 alkyl (meth)acrylate, of polyethylene glycol C10-C30 alkyl ether methacrylate containing 25 mol of ethylene oxide and of polyethylene glycol allyl ether containing 20 ethylene oxide units/polypropylene glycol containing 5 propylene oxide units, such as the product sold under the name Fixate® Plus Polymer by the company Lubrizol (INCI name: Polyacrylate-14).

The nonionic associative polymers that may be used in the invention are preferably chosen from:
(5) C12-C20 fatty alcohol ethers of hydroxyethylcellulose, for instance cetyl alcohol ethers of hydroxyethylcellulose (INCI name: Cetyl hydroxyethylcellulose) such as the products sold under the names Natrosol® Plus 330 CS Modified Hydroxyethylcellulose or Polysurf Modified Hydroxyethylcellulose sold by the company Ashland.
(6) nonionic associative polyurethane polyethers:
   Preferably, the nonionic associative polyurethane polyethers comprise at least two lipophilic hydrocarbon-based chains containing from 6 to 30 carbon atoms which are separated by a hydrophilic block, it being possible for the hydrocarbon-based chains to be side chains or chains at the end of the hydrophilic block. In particular, it is possible for one or more side chains to be envisaged. In addition, the polymer may comprise a hydrocarbon-based chain at one end or at both ends of a hydrophilic block.

The polyurethane polyethers may be multiblock, in particular in triblock form. The hydrophobic blocks may be at each end of the chain (for example: triblock copolymer having a hydrophilic central block) or distributed both at the ends and in the chain (for example, multiblock copolymer). These same polymers may also be graft polymers or star polymers.

The nonionic fatty-chain polyurethane polyethers may be triblock copolymers, the hydrophilic block of which is a polyoxyethylene chain comprising from 10 to 500 and preferably from 10 to 300 oxyethylene groups.

In a first variant, use may be made of a polyurethane polyether derived from the polycondensation of:
(i) a polyethylene glycol comprising from 120 to 250 mol of ethylene oxide,
(ii) a polyoxyethylenated monoalcohol comprising from 14 to 24 carbon atoms (especially decyltetradecyl alcohol or stearyl alcohol) comprising from 15 to 120 mol of ethylene oxide (especially from 20 to 100 OE), and
(iii) a diisocyanate containing from 6 to 20 carbon atoms, chosen especially from methylenebis(4-cyclohexyl isocyanate) (SMDI) and hexamethylene diisocyanate (HDI), and preferably HDI.

Such polyurethane polyethers are, for example:
- the polycondensate of polyethylene glycol containing 136 mol of ethylene oxide, stearyl alcohol polyoxyethylenated with 100 mol of ethylene oxide and hexamethylene diisocyanate (HDI) especially with a weight-average molecular weight (Mw) of 30 000 or 40 000 (INCI name: PEG-136/steareth-100/HDI copolymer), such as the product sold under the name Rheoluxe 811 or Nuvis FX 1100 (formerly Rheolate® FX 1100) by the company Elementis.
- the polycondensate of polyethylene glycol containing 240 mol of ethylene oxide, polyoxyethylenated decyltetradecyl alcohol containing 20 ethylene oxide units and hexamethylene diisocyanate (HDI), such as the product sold under the name Adekanol GT-730 by the company Adeka USA Corporation (INCI name: PEG-240/HDI copolymer bis-decyltetradeceth-20 ether).

In a second variant, use may be made of a polyurethane polyether derived from the polycondensation of:
(i) a polyethylene glycol comprising from 130 to 200 mol of ethylene oxide,
(ii) a monoalcohol comprising from 8 to 22 carbon atoms, especially decyl alcohol or stearyl alcohol, and
(iii) a diisocyanate containing from 6 to 20 carbon atoms, chosen especially from methylenebis(4-cyclohexyl isocyanate) (SMDI) and hexamethylene diisocyanate (HDI), and preferably SMDI.

Such polyurethane polyethers are, for example:
- a polycondensate of polyethylene glycol containing 150 or 180 mol of ethylene oxide, stearyl alcohol and methylenebis(4-cyclohexyl isocyanate) (SMDI) (INCI name: PEG-150/stearyl alcohol/SMDI copolymer), such as the product sold at 15% by weight in a matrix of maltodextrin (4%) and water (81%) under the name Aculyn 46 by the company The Dow Chemical Company;
- a polycondensate of polyethylene glycol containing 150 or 180 mol of ethylene oxide, decyl alcohol and methylenebis(4-cyclohexyl isocyanate) (SMDI) (INCI name: PEG-150/decyl alcohol/SMDI copolymer), such as the product sold at 35% by weight in a mixture of propylene glycol (39%) and water (26%) under the name Aculyn 44 Polymer by the company The Dow Chemical Company.

In a third variant, use may be made of a polycondensate of polyethylene glycol containing 200 mol of ethylene oxide, ether of polyethylene glycol containing 10 mol of ethylene oxide and/or of methylglucose, ether of polyethylene glycol containing 6 mol of ethylene oxide and of tridecyl alcohol, hexamethylene diisocyanate, and containing C16-C20 alcohol end groups, such as the product sold under the name Avalure® Flex 6 Polymer by the company Lubrizol (INCI name: Polyurethane-62).

Use is preferably made of the polyurethane polyethers described previously as examples.

Preferably, the associative polymers as described above have a weight-average molecular mass of less than 500 000 and even more preferentially of less than 100 000, preferably ranging from 5000 to 80 000, which may be measured via the methods known to those skilled in the art.

Preferably, the associative polymer is chosen from:
- methacrylic acid/methyl acrylate/polyoxyethylenated (40 OE) behenyl alcohol dimethyl meta-isopropenyl benzyl isocyanate, such as the product sold under the name Viscophobe DB 1000 by the company Amerchol (Dow Chemical) (INCl name: Polyacrylate-3);
- polycondensates of polyethylene glycol containing 150 or 180 mol of ethylene oxide, decyl alcohol and methylenebis(4-cyclohexyl isocyanate) (INCI name: PEG-150/decyl alcohol/SMDI copolymer), such as the product sold under the name Aculyn 44 Polymer by the company The Dow Chemical Company;
- polycondensates of polyethylene glycol containing 200 mol of ethylene oxide, ether of polyethylene glycol containing 10 mol of ethylene oxide and methylglucose, ether of polyethylene glycol containing 6 mol of ethylene oxide and of tridecyl alcohol, hexamethylene diisocyanate, and containing C16-C20 alcohol end groups, such as the product sold under the name Avalure® Flex 6 Polymer by the company Lubrizol (INCI name: Polyurethane-62).
- polycondensates of polyethylene glycol containing 240 mol of ethylene oxide, polyoxyethylenated tetradecyl alcohol containing 20 ethylene oxide units and hexamethylene diisocyanate (HDI), such as the product sold under the name Adekanol GT-730 by the company Adeka USA Corporation (INCI name: PEG-240/HDI copolymer bis-decyltetradeceth-20 ether).

Preferentially, the associative polymer is chosen from methacrylic acid/methyl acrylate/polyoxyethylenated (40 OE) behenyl alcohol dimethyl meta-isopropenyl benzyl isocyanate terpolymers, such as the product sold under the name Viscophobe DB 1000 by the company Amerchol (Dow Chemical) (INCI name: Polyacrylate-3).

The composition according to the invention may comprise a mixture of associative aqueous gelling agents as described previously.
According to one embodiment, the composition may comprise the combination:
i) of an acrylic terpolymer described in (3) previously, and preferably a terpolymer of methacrylic acid/methyl acrylate/condensate of dimethyl meta-isopropenyl benzyl isocyanate and of polyoxyethylenated (40 OE) behenyl alcohol (INCI name: Polyacrylate-3), such as the product sold in the form of an aqueous dispersion at 25% by weight, under the name Viscophobe DB 1000 by the company The Dow Chemical Company; and
ii) of an associative polymer comprising at least one hydrophilic unit of unsaturated ethylenic carboxylic acid type and at least one hydrophobic unit of (C₁₀-C₃₀)alkyl ester of unsaturated carboxylic acid type.
According to another embodiment, the composition may comprise the combination
i) of an acrylic terpolymer described in (3) previously, and preferably a terpolymer of methacrylic acid/methyl acrylate/condensate of dimethyl meta-isopropylenyl benzyl isocyanate and of polyoxyethylenated (40 OE) behenyl alcohol (INCI name: Polyacrylate-3), and ii) of a C12-C20 fatty alcohol ether of hydroxyethylcellulose, preferably a cetyl alcohol ether of hydroxyethylcellulose.

The associative hydrophilic gelling polymer may be present in the composition according to the invention in a content ranging from 0.1% to 2% by weight, relative to the total weight of the composition, preferably ranging from 0.2% to 1.5% by weight and preferentially ranging from 0.2% to 1% by weight.

### Additional hydrophilic gelling agent

Advantageously, the composition according to the invention may comprise at least one particular additional aqueous gelling agent as described below.

For the purposes of the present invention, the term "aqueous gelling agent", also referred to as a "hydrophilic gelling agent", means a compound that is capable of gelling the aqueous phase of the composition according to the invention.

The additional aqueous gelling agent is different from the associative polymer described previously.

The additional aqueous gelling agent is chosen from the following compounds:

### (1) crosslinked acrylic acid homopolymers.

The homopolymer may be crosslinked with a crosslinking agent, chosen especially from pentaerythrityl allyl ether, sucrose allyl ether and propylene allyl ether.

### Such polymers have the INCI name: Carbomer

Use may be made, for example, of the polymers sold by the company Lubrizol under the names Carbopol 980 or 981, or Carbopol Ultrez 10, or by the company 3V under the name Synthalen K or Synthalen L or Synthalen M.

### (2) carrageenans.

Carrageenans are sulfated polysaccharides constituting the cell walls of various red algae *(Rhodophyceae)* belonging especially to the *Gigartinacae, Hypneaceae, Furcellariaceae* and *Polyideaceae* families. Among these red algae, mention may be made, in a non-limiting manner, of *Kappaphycus alvarezii, Eucheuma denticulatum, Eucheuma spinosum, Chondrus crispus, Betaphycus gelatinum, Gigartina skottsbergii, Gigartina canaliculata, Sarcothalia crispata, Mazzaella laminaroides, Hypnea musciformis, Mastocarpus stellatus* and *Iridaea cordata.* They are generally obtained by hot aqueous extraction from natural strains of said algae.

These linear polymers, formed by disaccharide units, are composed of two D-galactopyranose units linked alternately by α(1,3) and β(1,4) bonds. They are highly sulfated polysaccharides (20-50%) and the α-D-galactopyranosyl residues may be in 3,6-anhydro form. Depending on the number and position of sulfate-ester groups on the repeating disaccharide of the molecule, several types of carrageenans are distinguished, namely: kappa-carrageenans, which bear one sulfate-ester group, iota-carrageenans, which bear two sulfate-ester groups, and lambda-carrageenans, which bear three sulfate-ester groups.

The carrageenans may be used in acid form or in salified form. Acceptable salts that may be mentioned, in a non-limiting manner, include the lithium, sodium, potassium, calcium, zinc and ammonium salts or the salts obtained with an organic base counterion, such as a primary, secondary or tertiary alkylamine, especially triethylamine or butylamine. This primary, secondary or tertiary alkylamine may comprise one or more nitrogen and/or oxygen atoms and may thus comprise, for example, one or more alcohol functions; mention may be made especially of 2-amino-2-methylpropanol, 2-dimethylamino-2-propanol and triethanolamine. Mention may also be made of lysine or 3-(dimethylamino)propylamine. These sulfated polysaccharides may also comprise a mixture of counterions from among those defined above in a non-limiting manner.

Carrageenans are sold especially by the company SEPPIC under the name Solagum®, by the company Gelymar under the names Carragel®, Carralact® and Carrasol®, by the company Cargill under the names Satiagel™ and Satiagum™, and by the company CP-Kelco under the names Genulacta®, Genugel® and Genuvisco®.

### lota-carrageenans are advantageously used.

### (3) alkali metal salts of acrylic acid homopolymers:

The homopolymer used according to the invention is chosen in particular from sodium polyacrylates and potassium polyacrylates. Sodium polyacrylate is preferably used.

The acrylic acid homopolymer may be advantageously neutralized to a degree ranging from 5% to 80%.

As sodium polyacrylate that can be used according to the invention, use may be made of those sold under the names Cosmedia SP® or Cosmedia SPL® by the company Cognis, or else Luvigel® EM sold by the company BASF.

Preferentially, the additional aqueous gelling agent is chosen from the alkali metal salts of acrylic acid homopolymers, and in particular from sodium polyacrylate homopolymers.

The additional hydrophilic gelling agent may be present in the composition according to the invention in a content ranging from 0.1% to 5% by weight, preferably ranging from 0.1% to 3% by weight, preferentially ranging from 0.1% to 2% by weight and better still ranging from 0.2% to 1.5% by weight, relative to the total weight of the composition.

### Oil

The composition according to the invention may comprise at least one oil.

The term "oil" means a fatty substance that is liquid at room temperature (25°C) and atmospheric pressure (760 mmHg, i.e. 10⁵ Pa). The oil may be volatile or non-volatile.

For the purposes of the invention, the term "volatile oil" refers to an oil that is capable of evaporating on contact with the skin or the keratin fibre in less than one hour, at room temperature and atmospheric pressure. The volatile oils of the invention are volatile cosmetic oils which are liquid at room temperature, with a non-zero vapour pressure, at room temperature and atmospheric pressure, ranging in particular from 0.13 Pa to 40 000 Pa (10⁻³ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" refers to an oil that remains on the skin or the keratin fibre at room temperature and atmospheric pressure for at least several hours, and that especially has a vapour pressure of less than 10⁻³ mmHg (0.13 Pa).

The oil may be chosen from any oil, which is preferably a physiologically acceptable oil, in particular mineral, animal, plant or synthetic oils; in particular volatile or non-volatile hydrocarbon-based oils and/or silicone oils and/or fluoro oils, and mixtures thereof.

More precisely, the term "hydrocarbon-based oil" means an oil mainly comprising carbon and hydrogen atoms and optionally one or more functions chosen from hydroxyl, ester, ether and carboxylic functions.

For the purposes of the present invention, the term "silicone oil" means an oil comprising at least one silicon atom, and especially at least one Si-O group.

For the purposes of the present invention, the term "fluoro oil" means an oil comprising at least one fluorine atom.
Hydrocarbon-based oils that may especially be mentioned include:
- hydrocarbon-based oils of plant origin such as triglycerides constituted by fatty acid esters of glycerol, the fatty acids of which may have chain lengths varying from C₄ to C₂₄, these chains possibly being linear or branched, and saturated or unsaturated; these oils are in particular heptanoic or octanoic acid triglycerides, or alternatively wheatgerm oil, sunflower oil, grapeseed oil, sesame seed oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, marrow oil, blackcurrant oil, evening primrose oil, millet oil, barley oil, quinoa oil, rye oil, safflower oil, candlenut oil, passion-flower oil and musk rose oil; shea butter; or else caprylic/capric acid triglycerides, such as those sold by the company Stéarineries Dubois or those sold under the names Miglyol® 810 and 812 by the company Cremer Oleo;
- linear or branched hydrocarbons, of mineral or synthetic origin, such as petroleum jelly, polydecenes, hydrogenated polyisobutene such as Parleam®, squalane, liquid paraffins, branched C₈-C₁₆ alkanes such as C₈-C₁₆ isoalkanes of petroleum origin (also known as isoparaffins), for instance isododecane (also known as 2,2,4,4,6-pentamethylheptane), isodecane, isohexadecane and, for example, the oils sold under the trade names Isopar® or Permethyl®;
- hydrocarbon-based esters of formula R₁COOR₂ in which R₁COO represents a carboxylic acid residue containing from 2 to 40 carbon atoms and R₂ represents a hydrocarbon-based chain, in particular a branched hydrocarbon-based chain containing from 1 to 40 carbon atoms, on condition that R₁ + R₂ ≥10, for instance cetostearyl octanoate, cetyl octanoate, tridecyl octanoate, isopropyl myristate, isopropyl palmitate, ethyl palmitate, C₁₂ to C₁₅ alcohol benzoates, hexyl laurate, diisopropyl adipate, isononyl isononanoate, 2-ethylhexyl palmitate, isostearyl isostearate, 2-hexyldecyl laurate, 2-octyldecyl palmitate, 2-octyldodecyl myristate, isodecyl neopentanoate, isotridecyl neopentanoate, isostearyl neopentanoate, 2-octyldecyl neopentanoate, isononyl isononanoate, isotridecyl isononanoate, octyl isononanoate, oleyl erucate; isocetyl stearate, isodecyl neopentanoate, isostearyl behenate, myristyl myristate;
- synthetic ethers containing from 10 to 40 carbon atoms, such as dicaprylyl ether;
- fatty alcohols that are liquid at room temperature, containing from 12 to 26 carbon atoms, for instance octyldodecanol, isostearyl alcohol, oleyl alcohol, 2-hexyldecanol, 2-butyloctanol and 2-undecylpentadecanol;
- higher fatty acids such as oleic acid, linoleic acid or linolenic acid, and mixtures thereof;
- silicone oils of volatile linear or cyclic silicones, especially those with a viscosity ≤ 5 centistokes (5 × 10⁻⁶ m²/s), and especially containing from 2 to 10 silicon atoms and preferably from 2 to 7 silicon atoms, these silicones optionally comprising alkyl or alkoxy groups containing from 1 to 10 carbon atoms. As volatile silicone oils that may be used in the invention, mention may be made especially of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.
- non-volatile silicone oils chosen especially from non-volatile polydimethylsiloxanes (PDMSs), polydimethylsiloxanes comprising alkyl or alkoxy groups, that are on the side and/or at the end of a silicone chain, the groups each containing from 2 to 24 carbon atoms, phenyl silicones, for instance phenyl trimethicones, phenyl dimethicones, trimethylsiloxyphenyl dimethicones, phenyltrimethylsiloxydiphenylsiloxanes, diphenyl dimethicones, diphenylmethyldiphenyltrisiloxanes, and mixtures thereof.

Advantageously, the composition according to the invention comprises an oil different from hydrocarbon-based oils of plant origin (triglycerides constituted of fatty acid esters of glycerol), especially such as those described previously, and optionally an additional oil chosen from hydrocarbon-based oils of plant origin (triglycerides constituted of fatty acid esters of glycerol).

The oil may be present in the composition according to the invention in a content ranging from 0% to 30% (or 0.1% to 30%) by weight, preferably ranging from 0% to 20% (or 0.1% to 20%) by weight, preferentially ranging from 0% to 15% (or 0.1% to 15%) by weight and better still ranging from 5% to 15% by weight, relative to the total weight of the composition.

When the composition according to the invention comprises an oil, the first and second solid fatty substances are advantageously present in a weight ratio of (first and second solid fatty substances)/oil ranging from 0.5 to 1.5, preferably ranging from 0.6 to 1.4, preferentially ranging from 0.7 to 1.3 and better still ranging from 0.9 to 1.2.

### Surfactant

The composition according to the invention may comprise at least one nonionic non-silicone surfactant, which is preferably an emulsifying surfactant.
The nonionic non-silicone surfactant may be chosen from:
- polyoxyethylenated C8-C30 fatty alcohols especially containing from 2 to 100 mol of ethylene oxide,
- C8-C30 fatty alcohol ethers of a sugar, in particular (C8-C30)alkyl (poly)glucosides,
- ethers of polyethylene glycol, especially containing from 20 to 120 ethylene oxide units, and of a C8-C30 fatty acid ester of glucose or of methylglucose,
- C₈-C₃₀ fatty acid esters of sorbitan,
- polyoxyethylenated C8-C30 fatty acid esters of sorbitan, especially containing from 2 to 30 mol of ethylene oxide,
- polyoxyethylenated C8-C30 fatty acid esters of sorbitan, especially containing from 2 to 100 mol of ethylene oxide,
- C8-C30 fatty acid monoesters or diesters of glycerol,
- polyglycerolated C8-C30 fatty acid esters, especially containing from 2 to 16 mol of glycerol,
- C8-C30 fatty acid esters of polyethylene glycol, especially containing from 2 to 200 ethylene oxide units,
- C8-C30 fatty acid esters of glucose or of (C1-C2)alkylglucose or of sucrose,
- and mixtures thereof.

Preferably, the nonionic non-silicone surfactant may be chosen from polyoxyethylenated C8-C30 fatty alcohols, especially containing from 2 to 100 mol of ethylene oxide, and (C8-C30)alkyl (poly)glucosides.

Preferentially, the nonionic non-silicone surfactant may be chosen from polyoxyethylenated C8-C30 fatty alcohols, especially containing from 2 to 100 mol of ethylene oxide.

The polyoxyethylenated C8-C30 fatty alcohols, especially containing from 2 to 100 mol of ethylene oxide, may be chosen from C12-C18 fatty alcohols, in particular polyoxyethylenated lauryl alcohol, cetyl alcohol, myristyl alcohol, stearyl alcohol and cetearyl alcohol (mixture of cetyl alcohol and stearyl alcohol), especially containing from 2 to 100 mol of ethylene oxide, such as:
cetyl alcohol polyoxyethylenated with 2 OE (Ceteth-2)
cetyl alcohol polyoxyethylenated with 6 OE (Ceteth-6)
cetyl alcohol polyoxyethylenated with 10 OE (Ceteth-10)
cetyl alcohol polyoxyethylenated with 20 OE (Ceteth-20)
cetyl alcohol polyoxyethylenated with 24 OE (Ceteth-24)
lauryl alcohol polyoxyethylenated with 2 OE (Laureth-2)
lauryl alcohol polyoxyethylenated with 3 OE (Laureth-3)
lauryl alcohol polyoxyethylenated with 4 OE (Laureth-4)
lauryl alcohol polyoxyethylenated with 7 OE (Laureth-7)
lauryl alcohol polyoxyethylenated with 9 OE (Laureth-9)
lauryl alcohol polyoxyethylenated with 10 OE (Laureth-10)
lauryl alcohol polyoxyethylenated with 12 OE (Laureth-12)
lauryl alcohol polyoxyethylenated with 21 OE (Laureth-21)
lauryl alcohol polyoxyethylenated with 23 OE (Laureth-23)
stearyl alcohol polyoxyethylenated with 2 OE (Steareth-2)
stearyl alcohol polyoxyethylenated with 10 OE (Steareth-10)
stearyl alcohol polyoxyethylenated with 20 OE (Steareth-20)
stearyl alcohol polyoxyethylenated with 21 OE (Steareth-21)
cetearyl alcohol polyoxyethylenated with 2 OE (Ceteareth-2)
cetearyl alcohol polyoxyethylenated with 10 OE (Ceteareth-10)
cetearyl alcohol polyoxyethylenated with 20 OE (Ceteareth-20)
cetearyl alcohol polyoxyethylenated with 25 OE (Ceteareth-25)
cetearyl alcohol polyoxyethylenated with 30 OE (Ceteareth-30)
cetearyl alcohol polyoxyethylenated with 40 OE (Ceteareth-40)
cetearyl alcohol polyoxyethylenated with 50 OE (Ceteareth-50)
cetearyl alcohol polyoxyethylenated with 60 OE (Ceteareth-60)
cetearyl alcohol polyoxyethylenated with 80 OE (Ceteareth-8)
cetearyl alcohol polyoxyethylenated with 100 OE (Ceteareth-100)

The nonionic surfactants may also be chosen from C8-C30 fatty alcohol ethers of a sugar, in particular (C8-C30)alkyl (poly)glucosides.

The alkyl (poly)glucoside may be chosen from a group comprising the compounds having the following general formula:

R₁O-(G)ₐ

in which R¹ denotes a linear or branched alkyl radical comprising from 8 to 30 carbon atoms and preferably from 8 to 24 carbon atoms, the G group denotes a sugar comprising from 5 to 6 carbon atoms and a is a number ranging from 1 to 10, and mixtures thereof.

The alkyl (poly)glucoside may be chosen especially from the group comprising C8-C22 fatty alcohol ethers or mixtures of ethers of glucose or of xylose, preferably of glucose.

The unit (or chain) derived from the fatty alcohol of the ethers may be chosen especially from caprylyl, capryl, decyl, lauryl, myristyl, cetyl (or palmityl), stearyl, octyldodecyl, arachidyl, behenyl and hexadecanoyl units, and mixtures thereof such as cetearyl.

In a particular embodiment, the alkyl (poly)glucoside is chosen from caprylyl/capryl glucoside, decyl glucoside, lauryl glucoside, myristyl glucoside, cetearyl glucoside, arachidyl glucoside, cocoyl glucoside, octyldodecyl glucoside, caprylyl/capryl xyloside, octyldodecyl xyloside, and a mixture thereof, preferably cetearyl glucoside and arachidyl glucoside.

Examples of alkyl (poly)glucosides that may be mentioned include caprylyl/capryl glucoside, for instance the product sold under the name Oramix CG 110 by the company SEPPIC, decyl glucoside sold, for example, under the names Plantaren 2000 by the company Henkel, Plantacare 2000 UP by the company Cognis, Mydol 10 by the company Kao, or Oramix NS 10 by the company SEPPIC, lauryl glucoside sold, for example, by the company Henkel under the name Plantaren 1200, cocoyl glucoside sold, for example, under the name Plantacare 818 UP by the company Cognis, cetearyl glucoside optionally as a mixture with cetearyl alcohol, sold, for example, under the name Montanov 68 by the company SEPPIC or under the name Xyliance by the company Soliance, under the name Tego Care CG90 by the company Evonik Goldschmidt and under the name Emulgade KE 3302 by the company Henkel, and also arachidyl glucoside, for example in the form of the mixture of arachidyl and behenyl alcohols and of arachidyl glucoside, sold under the name Montanov 202 by the company SEPPIC, the mixture of cocoyl polyglucoside and of cetyl and stearyl alcohols (35/65) sold, for example, under the name Montanov 82 by the company SEPPIC, octyldodecyl xyloside sold under the names Fluidanov 20X or Easynov by the company SEPPIC, myristyl glucoside, and especially in the form of a mixture with myristyl alcohol, for instance the product sold by the company SEPPIC under the name Montanov 14, mixtures of (C12-C20)alkyl glucosides especially as a mixture with C14 to C22 fatty alcohols, for instance the mixture sold under the name Montanov L by the company SEPPIC, and mixtures thereof.

According to a particular embodiment of the invention, the alkyl (poly)glucoside is chosen from arachidyl glucoside, especially as a mixture with arachidyl and behenyl alcohols, such as the product sold under the name Montanov 202 by the company SEPPIC, and mixtures thereof.

The ethers of polyethylene glycol, especially containing from 20 to 120 ethylene oxide units, and of a C8-C30 fatty acid ester of glucose or of methylglucose, may be chosen from:
the ether of polyethylene glycol containing about 20 mol of ethylene oxide and of the mixture of methylglucose monoester and diester of caprylic/capric acids (INCI name: PEG-20 methyl glucose sesquicaprylate/sesquicaprate)
the ether of polyethylene glycol containing about 80 mol of ethylene oxide and of the methylglucose ester of lauric acid (INCI name: PEG-80 methyl glucose laurate)
the ether of polyethylene glycol containing about 20 mol of ethylene oxide and of the mixture of methylglucose monoester and diester of lauric acid (INCI name: PEG-20 methyl glucose sesquilaurate)
the ether of polyethylene glycol containing about 20 mol of ethylene oxide and of the mixture of methylglucose monoester and diester of stearic acid (INCI name: PEG-20 methyl glucose sesquistearate)
the ether of polyethylene glycol containing about 20 mol of ethylene oxide and of the methylglucose diester of stearic acid (INCI name: PEG-20 methyl glucose distearate)
the ether of polyethylene glycol containing about 120 mol of ethylene oxide and of the methylglucose triester of stearic acid (INCI name: PEG-120 methyl glucose triisostearate)
the ether of polyethylene glycol containing about 120 mol of ethylene oxide and of the methylglucose diester of oleic acid (INCI name: PEG-120 methyl glucose dioleate)
the ether of polyethylene glycol containing about 120 mol of ethylene oxide and of the methylglucose triester of oleic acid (INCI name: PEG-120 methyl glucose trioleate).

The C₈-C₃₀ and preferably C₁₂-C₂₂ fatty acid esters (especially monoesters, diesters and triesters) of sorbitan may be chosen from:
sorbitan caprylate; sorbitan cocoate; sorbitan isostearate; sorbitan laurate; sorbitan oleate; sorbitan palmitate; sorbitan stearate
sorbitan diisostearate; sorbitan dioleate; sorbitan distearate;
sorbitan sesquicaprylate; sorbitan sesquiisostearate; sorbitan sesquioleate; sorbitan sesquistearate
sorbitan triisostearate; sorbitan trioleate; sorbitan tristearate.

The polyoxyethylenated C8-C30 (preferably C12-C18) fatty acid esters (especially monoesters, diesters and triesters) of sorbitan especially containing from 2 to 30 mol of ethylene oxide may be chosen from polyoxyethylenated esters of C12-C18 fatty acids, in particular lauric, myristic, cetylic or stearic acid, of sorbitan especially containing from 2 to 30 mol of ethylene oxide, such as:
polyoxyethylenated sorbitan monolaurate (4 OE) (Polysorbate-21)
polyoxyethylenated sorbitan monolaurate (20 OE) (Polysorbate-20)
polyoxyethylenated sorbitan monopalmitate (20 OE) (Polysorbate-40)
polyoxyethylenated sorbitan monostearate (20 OE) (Polysorbate-60)
polyoxyethylenated sorbitan monostearate (4 OE) (Polysorbate-61)
polyoxyethylenated sorbitan monooleate (20 OE) (Polysorbate-80)
polyoxyethylenated sorbitan monooleate (5 OE) (Polysorbate-81)
polyoxyethylenated sorbitan tristearate (20 OE) (Polysorbate-65)
polyoxyethylenated sorbitan trioleate (20 OE) (Polysorbate-85)

The polyoxyethylenated C8-C30 (preferably C12-C18) fatty acid esters (especially monoesters, diesters, triesters and tetraesters) of sorbitan, especially containing from 2 to 100 mol of ethylene oxide, may be chosen from polyoxyethylenated esters, especially containing from 2 to 100 mol of ethylene oxide, of C12-C18 fatty acids, in particular such as lauric, myristic, cetylic or stearic acid, and of sorbitan, such as:
the ester polyoxyethylenated with 20 OE of sorbitan and of cocoic acid (PEG-20 sorbitan cocoate);
the diester polyoxyethylenated with 40 OE of isostearic acid and of sorbitan (PEG-40 sorbitan diisostearate);
the polyoxyethylenated esters (especially containing from 2 to 20 OE) of sorbitan and of isostearic acid (such as PEG-2 sorbitan isostearate; PEG-5 sorbitan isostearate; PEG-20 sorbitan isostearate such as the product sold under the name Nikkol TI 10 V by the company Nikkol);
the polyoxyethylenated esters (especially containing from 2 to 40 OE) of sorbitan and of lauric acid (such as PEG-10 sorbitan laurate; PEG-40 sorbitan laurate);
the polyoxyethylenated esters (especially containing from 2 to 40 OE) of sorbitan and of oleic acid containing 10 oxyethylene groups (such as PEG-6 sorbitan oleate; PEG-20 sorbitan oleate);
the polyoxyethylenated esters (especially containing from 3 to 60 OE) of sorbitan and of stearic acid (such as PEG-3 sorbitan stearate; PEG-4 sorbitan stearate; PEG-6 sorbitan stearate; PEG-40 sorbitan stearate; PEG-60 sorbitan stearate);
the polyoxyethylenated tetraesters (especially containing from 30 to 60 OE) of sorbitan and of oleic acid (such as PEG-30 sorbitan tetraoleate; PEG-40 sorbitan tetraoleate; PEG-60 sorbitan tetraoleate);
the polyoxyethylenated triesters (especially containing from 4 to 60 OE) of sorbitan and of (iso)stearic acid (such as PEG-4 sorbitan triisostearate; PEG-20 sorbitan triisostearate; PEG-3 sorbitan tristearate).

The C8-C30 (preferably C12-C18) fatty acid monoesters of glycerol may be chosen from glyceryl caprylate, glyceryl caprate, glyceryl laurate, glyceryl myristate, glyceryl palmitate, glyceryl isostearate (Peceol Isostéarique from Gattefosse), glyceryl stearate, glyceryl oleate, glyceryl cocoate, glyceryl behenate (Compritol 888 ATO from Gattefosse), glyceryl arachidate; glycerol diesters such as glyceryl dilaurate, glyceryl dimyristate, glyceryl dipalmitate, glyceryl diisostearate, glyceryl distearate, glyceryl dioleate, glyceryl dibehenate, glyceryl diarachidate.

The polyglycerolated C8-C30 fatty acid esters especially containing from 2 to 16 mol of glycerol may be chosen from polyglycerolated esters of C12-C18 fatty acids, in particular lauric, myristic, palmitic, stearic or isostearic acid, especially containing from 2 to 16 mol of glycerol, such as:
polyglyceryl-2 laurate, polyglyceryl-3 laurate, polyglyceryl-4 laurate, polyglyceryl-5 laurate, polyglyceryl-6 laurate, polyglyceryl-10 laurate;
polyglyceryl-2 myristate, polyglyceryl-3 myristate, polyglyceryl-4 myristate, polyglyceryl-5 myristate, polyglyceryl-6 myristate, polyglyceryl-10 myristate;
polyglyceryl-2 palmitate, polyglyceryl-3 palmitate, polyglyceryl-6 palmitate, polyglyceryl-10 palmitate;
polyglyceryl-2 isostearate, polyglyceryl-3 isostearate, polyglyceryl-4 isostearate, polyglyceryl-5 isostearate, polyglyceryl-6 isostearate, polyglyceryl-10 isostearate;
polyglyceryl-2 stearate, polyglyceryl-3 stearate, polyglyceryl-4 stearate, polyglyceryl-5 stearate, polyglyceryl-6 stearate, polyglyceryl-8 stearate, polyglyceryl-10 stearate.

The C8-C30 (preferably C12-C18) fatty acid esters of polyethylene glycol, especially containing from 2 to 200 ethylene oxide units, may be chosen from:
PEG-8 behenate; PEG-8 caprylate; PEG-8 caprate; PEG-6 caprylate/caprate; PEG-8 caprylate/caprate; PEG-5 cocoate; PEG-8 cocoate; PEG-9 cocoate; PEG-10 cocoate; PEG-15 cocoate; PEG-4 ethylhexanoate; PEG-5 ethylhexanoate; PEG-13 ethylhexanoate; PEG-2 isostearate; PEG-4 isostearate; PEG-6 isostearate; PEG-8 isostearate; PEG-10 isostearate; PEG-12 isostearate; PEG-20 isostearate; PEG-30 isostearate; PEG-40 isostearate; PEG-2 laurate; PEG-4 laurate; PEG-6 laurate; PEG-8 laurate; PEG-9 laurate; PEG-10 laurate; PEG-12 laurate; PEG-14 laurate;PEG-20 laurate; PEG-32 laurate; PEG-75 laurate; PEG-150 laurate; PEG-2 oleate; PEG-4 oleate; PEG-6 oleate; PEG-8 oleate; PEG-10 oleate; PEG-12 oleate; PEG-14 oleate; PEG-16 oleate; PEG-20 oleate; PEG-32 oleate; PEG-75 oleate; PEG-150 oleate; PEG-6 palmitate; PEG-18 palmitate; PEG-20 palmitate; PEG-2 stearate; PEG-4 stearate; PEG-6 stearate; PEG-8 stearate; PEG-10 stearate; PEG-14 stearate; PEG-18 stearate; PEG-20 stearate; PEG-25 stearate; PEG-30 stearate; PEG-35 stearate; PEG-40 stearate; PEG-45 stearate; PEG-50 stearate; PEG-55 stearate; PEG-75 stearate; PEG-80 stearate; PEG-90 stearate; PEG-100 stearate; PEG-120 stearate; PEG-150 stearate.

The C8-C30 (preferably C12-C18) fatty acid esters of glucose or of (C1-C2)alkylglucose or of sucrose may be chosen from glucose palmitate, (C1-C2)alkylglucose sesquistearates, for instance methylglucose sesquistearate, (C1-C2)alkylglucose palmitates, for instance methylglucose palmitate or ethylglucose palmitate, fatty esters of methylglucoside and more especially the diester of methylglucoside and of oleic acid (INCI name: Methyl glucose dioleate); the ester of methylglucoside and of isostearic acid (INCI name: Methyl glucose isostearate); the ester of methylglucoside and of lauric acid (INCI name: Methyl glucose laurate); the mixture of the monoester and diester of methylglucoside and of isostearic acid (INCI name: methyl glucose sesquiisostearate); the mixture of the monoester and diester of methylglucoside and of stearic acid (INCI name: methyl glucose sesquistearate) and in particular the product sold under the name Glucate SS by the company Amerchol, and mixtures thereof.
Sucrose esters that may be mentioned include sucrose cocoate; sucrose dilaurate; sucrose distearate; sucrose laurate; sucrose myristate; sucrose oleate; sucrose palmitate and sucrose stearate.

The nonionic non-silicone surfactant may be present in the composition according to the invention in a content ranging from 0.1% to 4% by weight, preferably from 0.1% to 3% by weight, better still from 0.4% to 2.5% by weight and even better still ranging from 0.4% to 2% by weight, relative to the total weight of the composition.

### Aqueous phase

The composition according to the invention comprises an aqueous phase.

The aqueous phase comprises at least water. Water may be present in the composition according to the invention in a content ranging from 25% to 90% by weight, ranging from 30% to 80% by weight and better still from 40% to 70% by weight, relative to the total weight of the composition.

The aqueous phase may comprise at least one organic solvent that is water-miscible at room temperature (25°C), for instance linear or branched monoalcohols containing from 2 to 6 carbon atoms, such as ethanol, propanol, isopropanol, butantol, isobutanol, pentanol or hexanol; polyols especially containing from 2 to 20 carbon atoms, preferably from 2 to 6 carbon atoms, such as glycerol, propylene glycol, isoprene glycol, butylene glycol, pentylene glycol, hexylene glycol, sorbitol and polyethylene glycols containing from 2 to 200 ethylene units, and mixtures thereof. The water-miscible organic solvent may be present in the composition in a content ranging from 0.1% to 45% by weight relative to the total weight of the composition.

### Organopolysiloxane elastomer

The composition of the invention may comprise at least one non-emulsifying elastomeric organopolysiloxane, also referred to in the rest of the description as a "silicone elastomer". The term "elastomer" means a deformable, flexible solid material having viscoelastic properties and especially the consistency of a sponge or of a supple sphere. Its modulus of elasticity is such that this material withstands deformation and has limited stretchability and contractibility. This material is capable of regaining its original shape after stretching. This elastomer is formed from polymer chains of high molecular weight, the mobility of which is limited by a uniform network of crosslinking points.

According to a particular embodiment of the invention, the elastomeric organopolysiloxane(s) used in the composition are partially or totally crosslinked. They may then be in the form of particles. These particles may have any form and may, for example, be spherical, flat or amorphous.

When they are included in an oily phase, these elastomeric organopolysiloxanes become transformed, depending on the amount of oily phase used, into a product of spongy appearance when they are used in the presence of small amounts of oily phase, or into a homogeneous gel in the presence of larger amounts of oily phase. The gelation of the oily phase with these elastomers may be total or partial.
Thus, the elastomers of the invention may be conveyed in the form of an anhydrous gel constituted of an elastomeric organopolysiloxane and of an oily phase. The oily phase used in the manufacture of the anhydrous gel of elastomeric organopolysiloxane contains one or more oils that are liquid at room temperature (25°C) chosen from hydrocarbon-based oils and/or silicone oils. Advantageously, the oily phase is a liquid silicone phase, containing one or more oils chosen from linear-chain or cyclic-chain polydimethylsiloxanes, which are liquid at room temperature, optionally comprising an alkyl or aryl chain that is on the side or at the end of the chain, the alkyl chain containing from 1 to 6 carbon atoms.

The elastomeric organopolysiloxanes used according to the invention may be chosen from the crosslinked polymers described in patent application EP-A-0 295 886 and from those described in patent US-A-5 266 321. They may be emulsifying or non-emulsifying.

The term "non-emulsifying elastomeric organopolysiloxanes" refers to a silicone elastomer not comprising a hydrophilic chain, such as a (poly)oxyalkylene or (poly)glycerol chain.

The organic groups bonded to the silicon atoms of compound (A1) may be alkyl groups containing from 1 to 18 carbon atoms, such as methyl, ethyl, propyl, butyl, octyl, decyl, dodecyl (or lauryl), myristyl, cetyl or stearyl; substituted alkyl groups such as 2-phenylethyl, 2-phenylpropyl or 3,3,3-trifluoropropyl; aryl groups such as phenyl, tolyl or xylyl; substituted aryl groups such as phenylethyl; and substituted monovalent hydrocarbon-based groups such as an epoxy group, a carboxylate ester group or a mercapto group.

Compound (A1) may thus be chosen from trimethylsiloxy-terminated methylhydropolysiloxanes, trimethylsiloxy-terminated dimethylsiloxane/methylhydrosiloxane copolymers, dimethylsiloxane/methylhydrosiloxane cyclic copolymers, and trimethylsiloxy-terminated dimethylsiloxane/methylhydrosiloxane/laurylmethylsiloxane copolymers. Compound (C1) is the catalyst for the crosslinking reaction, and is in particular chloroplatinic acid, chloroplatinic acid-olefin complexes, chloroplatinic acid-alkenylsiloxane complexes, chloroplatinic acid-diketone complexes, platinum black and platinum on a support.

The term "non-emulsifying elastomeric organopolysiloxanes" refers to organopolysiloxane elastomers not containing a hydrophilic chain such as polyoxyalkylene or polyglycerol units.

According to a particular embodiment of the invention, the non-emulsifying silicone elastomer(s) are elastomeric crosslinked organopolysiloxanes which may be obtained via a crosslinking addition reaction of diorganopolysiloxane containing at least one hydrogen bonded to silicon and of diorganopolysiloxane containing ethylenically unsaturated groups bonded to silicon, especially in the presence of a platinum catalyst; or via a dehydrogenation crosslinking condensation reaction between a hydroxy-terminated diorganopolysiloxane and a diorganopolysiloxane containing at least one hydrogen bonded to silicon, especially in the presence of an organotin compound; or via a crosslinking condensation reaction of a hydroxy-terminated diorganopolysiloxane and of a hydrolysable organopolysilane; or via thermal crosslinking of organopolysiloxane, especially in the presence of an organoperoxide catalyst; or via crosslinking of organopolysiloxane by high-energy radiation such as gamma rays, ultraviolet rays or an electron beam.

Preferably, the elastomeric crosslinked organopolysiloxane is obtained by a crosslinking addition reaction (A) of a diorganopolysiloxane containing at least two hydrogen atoms each bonded to a different silicon atom, and (B) of a diorganopolysiloxane containing at least two ethylenically unsaturated groups bonded to silicon, especially in the presence (C) of a platinum catalyst, for instance as described in patent application EP-A-295 886.

In particular, the organopolysiloxane may be obtained by reaction of dimethylvinylsiloxy-terminated dimethylpolysiloxane and of trimethylsiloxy-terminated methylhydropolysiloxane, in the presence of a platinum catalyst.

Compound (A) is the base reagent for the formation of elastomeric organopolysiloxane, and the crosslinking reaction is performed by addition reaction of compound (A) with compound (B) in the presence of catalyst (C).

Compound (A) is advantageously a diorganopolysiloxane containing at least two lower (for example C2-C4) alkenyl groups; the lower alkenyl group may be chosen from vinyl, allyl and propenyl groups. These lower alkenyl groups may be located in any position on the organopolysiloxane molecule, but are preferably located at the ends of the organopolysiloxane molecule. The organopolysiloxane (A) may have a branched chain, linear chain, cyclic or network structure, but the linear chain structure is preferred. Compound (A) may have a viscosity ranging from the liquid state to the gum state. Preferably, compound (A) has a viscosity of at least 100 centistokes at 25°C.

The organopolysiloxanes (A) may be chosen from methylvinylsiloxanes, methylvinylsiloxane/dimethylsiloxane copolymers, dimethylvinylsiloxy-terminated dimethylpolysiloxanes, dimethylvinylsiloxy-terminated dimethylsiloxane/methylphenylsiloxane copolymers, dimethylvinylsiloxy-terminated dimethylsiloxane/diphenylsiloxane/methylvinylsiloxane copolymers, trimethylsiloxy-terminated dimethylsiloxane/methylvinylsiloxane copolymers, trimethylsiloxy-terminated dimethylsiloxane/methylphenylsiloxane/methylvinylsiloxane copolymers, dimethylvinylsiloxy-terminated methyl(3,3,3-trifluoropropyl)polysiloxanes, and dimethylvinylsiloxy-terminated dimethylsiloxane/methyl(3,3,3-trifluoropropyl)siloxane copolymers.

Compound (B) is in particular an organopolysiloxane containing at least 2 hydrogens bonded to silicon in each molecule and is thus the crosslinking agent for compound (A).

Advantageously, the sum of the number of ethylenic groups per molecule in compound (A) and the number of hydrogen atoms bonded to silicon per molecule in compound (B) is at least 4.

Compound (B) may be in any molecular structure, especially in a linear chain, branched chain or cyclic structure.

Compound (B) may have a viscosity at 25°C ranging from 1 to 50 000 centistokes, especially so as to be miscible with compound (A).

It is advantageous for compound (B) to be added in an amount such that the molecular ratio between the total amount of hydrogen atoms bonded to silicon in compound (B) and the total amount of all the ethylenically unsaturated groups in compound (A) is in the range from 1/1 to 20/1.

Compound (B) may be chosen from trimethylsiloxy-terminated methylhydropolysiloxanes, trimethylsiloxy-terminated dimethylsiloxane/methylhydrosiloxane copolymers and dimethylsiloxane/methylhydrosiloxane cyclic copolymers.

Compound (C) is the catalyst for the crosslinking reaction, and is especially chloroplatinic acid, chloroplatinic acid-olefin complexes, chloroplatinic acid-alkenylsiloxane complexes, chloroplatinic acid-diketone complexes, platinum black and platinum on a support.

Catalyst (C) is preferably added in an amount of from 0.1 to 1000 parts by weight and better still from 1 to 100 parts by weight, as clean platinum metal, per 1000 parts by weight of the total amount of compounds (A) and (B).

Other organic groups may be bonded to silicon in the organopolysiloxanes (A) and (B) described previously, for instance alkyl groups such as methyl, ethyl, propyl, butyl or octyl; substituted alkyl groups such as 2-phenylethyl, 2-phenylpropyl or 3,3,3-trifluoropropyl; aryl groups such as phenyl, tolyl or xylyl; substituted aryl groups such as phenylethyl; and substituted monovalent hydrocarbon-based groups such as an epoxy group, a carboxylate ester group or a mercapto group.

The non-emulsifying silicone elastomer is generally mixed with at least one hydrocarbon-based oil and/or one silicone oil to form a gel. In these gels, the non-emulsifying elastomer is in the form of non-spherical particles.

The non-emulsifying elastomeric organopolysiloxanes used in the composition of the invention may be, for example, those sold under the names KSG 6 by the company Shin-Etsu; Trefil E-505C or Trefil E-506C by the company Dow Corning; Gransil (SR-CYC, SR DMF10, SR-DC556) by the company Grant Industries, or those sold in the form of already-constituted gels: KSG 15, KSG 16, KSG 17, KSG 18, KSG 26A, KSG 26B, KSG-31, KSG-32, KSG-33, KSG-41, KSG-42, KSG-43 and KSG-44 from the company Shin-Etsu; Gransil SR 5CYC Gel, Gransil SR DMF 10 Gel, Gransil SR DC556 Gel and Gransil RPS from Grant Industries; 1229-02-167 and 1229-02-168 from the company General Electric.

Use may also be made of silicone elastomers having the INCI name dimethicone/vinyl dimethicone copolymer (or polysilicone-11) as a mixture with a cyclic silicone oil. Examples that may be mentioned include the mixture of crosslinked organopolysiloxane/cyclopentasiloxane or a mixture of crosslinked organopolysiloxane/cyclohexasiloxane, for instance Gransil RPS D5 or Gransil RPS D6 from the company Grant Industries.

Mention may also be made of the elastomers sold under the references DC 9040, DC 9041, DC 9509, DC 9505 and DC 9506 by the company Dow Corning. Use may also be made of a mixture of silicone elastomers, and especially a mixture of these commercial products.

The non-emulsifying elastomeric organopolysiloxane may be present in the composition according to the invention in an (active material) content ranging from 0 to 2% (or 0.1% to 2%), preferably ranging from 0 to 1.5% (0.1% to 1.5%) by weight and preferentially ranging from 0 to 1% by weight, relative to the total weight of the composition.

The composition of the invention may comprise additives that are common in the cosmetic and/or dermatological fields, such as moisturizers, emollients, hydrophilic or lipophilic active agents, free-radical scavengers, sequestrants, antioxidants, preserving agents, acidifying or basifying agents, fragrances, film-forming agents, fillers, dyestuffs, UV-screening agents, and mixtures thereof.

According to a particular embodiment, the composition according to the invention has a compact texture and thus has a particular rheological profile, due to the presence of the solid fatty substances and of the associative aqueous gelling polymer.

Advantageously, the composition according to the invention has a hardness, at 25°C, ranging from 1 to 30 kPa (better still from 2 to 30 kPa or from 3 to 30 kPa), preferably ranging from 1 to 15 kPa (better still from 2 to 15 kPa or from 3 to 15 kPa) and preferentially ranging from 1 to 10 kPa (better still from 2 to 10 kPa or from 3 to 10 kPa).

The hardness of the composition is measured at 25°C according to the following protocol: The composition is packaged in a levelled-off 15 ml glass cosmetics jar.
The compression force is measured with a TA XT2i texturometer and the Texture Expert Exceed software. The spindle used is the stainless-steel P/6 mm cylinder, with the following parameters:
Spindle descent speed: 1 mm/s
Measuring speed: 1 mm/s
Penetration depth: 5 mm
Spindle withdrawal speed: 10 mm/s
The measurements are taken on two samples.
The value obtained is the maximum force Fₘₐₓ expressed in grams (g).
The hardness is calculated according to the equation: D = Fₘₐₓ / S
D: hardness in MPa
Fₘₐₓ: Maximum force measured, expressed in newtons (1N equals 101.97 g)
S: Contact area of the spindle πr² (mm²) (i.e. 28.26 mm²)

The invention also relates to a process for preparing the composition according to the invention, comprising the following steps:
the aqueous phase containing the water, the associative gelling polymer, optionally the additional hydrophilic thickening polymer and the hydrophilic additives is homogenized at a temperature ranging from 60 to 90°C, preferably from 70 to 75°C;
the fatty phase, comprising the first and second solid fatty substances and optionally comprising the oil, the non-emulsifying elastomeric organopolysiloxane and the lipophilic additives, is homogenized at a temperature ranging from 75 to 100°C, preferably from 85 to 90°C;
the homogenized fatty phase is then introduced into the homogenized aqueous phase at a temperature ranging from 60 to 90°C, preferably from 70 to 75°C;
the mixture is then allowed to cool to room temperature.

### Examples

Unless otherwise mentioned, the amounts indicated are mass amounts relative to the total weight of the composition.

In the compositions described below, the following solid fatty substances were used, having the hardness and melting point indicated (listed in increasing order of hardness):
Mango butter sold under the name Trivent Mango Butter by the company Alzo (hardness = 0.26 MPa; m.p. = 36.1°C)
Mixture of mimosa, jojoba and sunflower plant waxes sold under the name Acticire by the company Gattefosse (hardness = 0.49 MPa; m.p. = 38.2°C)
Cocoa butter sold under the name CT Cocoa Butter Deodorized by the company Dutch Cocoa BV (hardness = 1.05 MPa; m.p. = 32°C)
Hydrogenated jojoba oil butter sold under the name Iso Jojoba 50 by the company Desert Whale (hardness = 1.14 MPa; m.p. = 30°C)
Paraffin wax sold under the name Sasol Wax 5603 by the company Sasol (hardness = 7.4 MPa; m.p. = 58.1°C)
Ozokerite wax sold under the name Paracera ARFB by the company Paramelt (hardness = 8.8 MPa; m.p. = 55.8°C)
Stearyl alcohol (hardness = 9.27 MPa; m.p. = 62°C)
Polystearyl acrylate sold under the name Intelimer IPA 13-1 NG Polymer by the company Air Products and Chemicals (hardness = 9.5 MPa; m.p. = 47°C)
Candelilla wax sold under the name Candelilla Wax SP 75 G by the company Strahl & Pitsch (hardness = 9.6 MPa; m.p. = 64.9°C)
Behenyl alcohol (hardness = 10.6 MPa; m.p. = 67.4°C)
Carnauba wax sold under the name Cerauba T3 by the company Baerlocher (hardness = 10.7 MPa; m.p. = 82.3°C)

The associative aqueous gelling agents used are as follows:
Rheoluxe 811 (Elementis): PEG-136/Steareth-100/HDI Copolymer
Viscophobe DB 1000 (Dow Chemical Company): Terpolymer of methacrylic acid/methyl acrylate/condensate of dimethyl meta-isopropenyl benzyl isocyanate and of polyoxyethylenated (40 OE) behenyl alcohol (INCI name: Polyacrylate-3)
Aculyn 44 Polymer (Dow Chemical Company): polycondensate of polyethylene glycol comprising 150 or 180 mol of ethylene oxide, of decyl alcohol and of methylenebis(4-cyclohexyl isocyanate) at 35% by weight in a mixture of propylene glycol (39%) and water (26%) (INCI name: PEG-150/Decyl Alcohol/SMDI Copolymer)
Adekanol GT- 730 (Adeka USA Corporation): polycondensate of polyethylene glycol containing 240 mol of ethylene oxide, polyoxyethylenated decyltetradecyl alcohol containing 20 ethylene oxide units and hexamethylene diisocyanate (INCI name: PEG-240/HDI Copolymer Bis-Decyltetradeceth-20 Ether)
Polysurf modified hydroxyethylcellulose (Ashland): cetylhydroxyethylcellulose
Pemulen TR-1 Polymer (Noveon): acrylic acid/stearyl methacrylate copolymer polymerized in an ethyl acetate/cyclohexane mixture

### Examples 1 and 2:

The following two facial care compositions were prepared (weight contents):

| | | **Example 1** | **Example 2** |
|---|---|---|---|
| | Water | qs 100 | qs 100 |
| Aqueous phase | Glycerol | 7 | 7 |
| | Preserving agents | 1.2 | 1.2 |
| | Sequestrant | 0.1 | 0.1 |
| | PEG-136/steareth-100/HDI copolymer (Rheoluxe 811 from Elementis) | 0.25 | 0.5 |
| | Sodium polyacrylate (Cosmedia SP from Cognis) | 0.8 | 0.8 |
| | Boron nitride | 2 | 2 |
| Fatty phase | Mixture of arachidyl glucoside and of arachidyl and behenyl alcohols (15/55/30) (Montanov 202 from SEPPIC) | 3 | 3 |
| | Polystearyl acrylate | 1 | 1 |
| | Isostearyl neopentanoate | 5 | 5 |
| | Candelilla wax | - | 3 |
| | Cocoa butter | 4 | 4 |
| | Carnauba wax | 4 | - |
| | Dimethicone (Xiameter PMX-200 Silicone Fluid 10cS from Dow Corning) | 5 | 5 |
| | Mixture of crosslinked polydimethylsiloxane and of polydimethylsiloxane (15.5/84.5) (Dow Corning 9041 Silicone Elastomer Blend from Dow Corning) | 5 | 5 |
| | TOTAL | 100 | 100 |
| | **Hardness (kPa)** | **13.19** | **25.33** |

The aqueous phase was homogenized at a temperature of about 70-75°C. The fatty phase was homogenized at a temperature of about 85-90°C. The fatty phase was then introduced into the aqueous phase, such that the emulsification temperature was between 70 and 75°C, over 10 minutes. The thickener was then added. The mixture was then allowed to cool to room temperature.
The compositions obtained are stable after storage for two months, at room temperature (25°C) and at 45°C; they have a compact texture in accordance with their hardness.
Compositions 1 and 2 were evaluated by a panel of 12 women from 40 to 60 years old who were regular users of anti-ageing care products. These women applied compositions 1 and 2 to the skin of their face under the usual application conditions.
The panel judged that the compositions tested have the following cosmetic properties:
On application, compositions 1 and 2 are easy to spread and penetrate quickly. The textures are non-tacky and afford a sensation of softness on application.
After application of compositions 1 and 2 to the face, no sensation of tautness or tackiness was experienced.
After 3 days of use, compositions 1 and 2 are judged to be comfortable.
For composition 1, the majority of the panel found an anti-ageing effect (smooth, taut skin) and a skin-firming effect.
For composition 2, the majority of the panel found that the facial pores are tightened, and also found an anti-ageing effect (taut, reinvigorated, tonified, firmed skin, attenuation of fine lines).

### Example 3:

The following facial care composition was prepared:

| | |
|---|---|
| Isostearyl neopentanoate | 5% |
| Polystearyl acrylate | 1% |
| Behenyl alcohol | 1.2% |
| Carnauba wax | 2% |
| Paraffin wax | 2% |
| Dimethicone (Xiameter PMX-200 Silicone Fluid 10cS from Dow Corning) | 5% |
| Mixture of dimethicone and dimethicone crosspolymer (Dow Corning 9041 Silicone Elastomer Blend from Dow Corning) | 5% |
| Boron nitride | 2% |
| Oxyethylenated (25 OE) cetylstearyl alcohol | 1.8% |
| Acticire | 4% |
| Methacrylic acid/methyl acrylate/ ethoxylated alcohol dimethyl meta-isopropenyl benzyl isocyanate at 23% AM in water (Viscophobe DB 1000 from Amerchol) | 2.17% |
| Sodium polyacrylate (Cosmedia SP from Cognis) | 0.8% |
| Glycerol | 7% |
| Sequestrant | 0.1% |
| Preserving agents | qs |
| Water | qs 100% |

The composition obtained has a hardness of 6.59 kPa.

The composition obtained is stable after storage for two months, at room temperature (25°C) and at 45°C; it has a compact texture in accordance with its hardness.
The composition was evaluated by a panel of 12 women over 55 years old to obtain the perception by the panel of the sensation on use and the effects perceived on the skin.
The panel judged that the composition tested has the following cosmetic properties:
The composition has a compact texture: when the composition packaged in a jar is taken up with a finger, the finger does not enter the composition but remains in contact with the surface of the composition.
On application to facial skin, the composition penetrates rapidly into the skin, despite its compactness. After application, the skin is enveloped with a covering film.
Capacity for covering the surface of the skin, especially giving rise to tightening of the pores, which maintains the skin at the surface, affording a sensation of containment.
The skin feels like it is enveloped with a supple, invisible covering film.
The user has a sensation of restructuring and redensifying of the skin tissues.

### Examples 4 to 11:

The following skincare compositions were prepared:

| **Example** | **4** | **5** | **6** | **7** | **8** | **9** | **10** | **11** |
|---|---|---|---|---|---|---|---|---|
| Isostearyl neopentanoate | 5 | 5 | 5 | 5 | 5 | 5 | 5 | 5 |
| Carnauba wax | 2 | 2 | 2 | 2 | 2 | 2 | 2 | 2 |
| Paraffin wax | 2 | 2 | 2 | - | - | - | - | - |
| Ozokerite wax | - | 1.2 | - | 2 | 2 | 2 | 2 | 2 |
| Behenyl alcohol | 1.2 | - | 1.2 | 1.2 | 1.2 | 1.2 | 1.2 | 1.2 |
| Mango butter | 4 | 4 | - | - | - | | | |
| Acticire | | | | - | 4 | | | |
| Hydrogenated jojoba oil butter | | | 4 | 4 | - | | | |
| Cocoa butter | | | | | | 4 | 4 | 4 |
| Polystearyl acrylate | 1 | 1 | 1 | 1 | 1 | 1 | 1 | 1 |
| Dimethicone (Xiameter PMX-200 Silicone Fluid 10cS from Dow Corning) | 5 | 5 | 5 | 5 | 5 | 5 | 5 | 5 |
| Mixture of dimethicone and dimethicone crosspolymer (Dow Corning 9041 Silicone Elastomer Blend from Dow Corning) | 5 | 5 | 5 | 5 | 5 | 5 | 5 | 5 |
| Boron nitride | 2 | 2 | 2 | 2 | 2 | 2 | 2 | 2 |
| Oxyethylenated (25 OE) cetylstearyl alcohol | 1.8 | 1.8 | 1.8 | 1.8 | 1.8 | 1.8 | 1.8 | 1.8 |
| Sodium polyacrylate (Cosmedia SP from Cognis) | 0.8 | 0.8 | 0.8 | 0.8 | 0.8 | 0.8 | 0.8 | 0.8 |
| Viscophobe DB 1000 | 2.17 | 2.17 | 2.17 | 2.17 | 2.17 | | | |
| Aculyn 44 | | | | | | 1.43 | | |
| Adekanol GT- 730 | | | | | | | 0.5 | |
| PUR-62 | | | | | | | | 1 |
| Glycerol | 7 | 7 | 7 | 7 | 7 | 7 | 7 | 7 |
| Triethanolamine | 0.175 | 0.175 | 0.175 | 0.175 | 0.175 | 0.175 | 0.175 | 0.175 |
| Sequestrant | 0.1 | 0.1 | 0.1 | 0.1 | 0.1 | 0.1 | 0.1 | 0.1 |
| Preserving agents | qs | qs | qs | qs | qs | qs | qs | qs |
| Water | qs 100% | qs 100% | qs 100% | qs 100% | qs 100% | qs 100% | qs 100% | qs 100% |
| **Hardness (kPa)** | **6.94** | **6.94** | **6.59** | **8.33** | **7.29** | **1.39** | **5.55** | **7.98** |

The compositions obtained are stable after storage for two months, at room temperature (25°C) and at 45°C; compositions 4 to 8, 10 and 11 have a compact texture in accordance with their hardness.

The compositions spread and penetrate easily on/into the skin. After application, the applied compositions are not tacky or greasy and have a soft, pleasant feel, without any sensation of heaviness.

After application to facial skin, the compositions afford a covering on the surface of the skin, which maintains the skin at the surface, affording a sensation of containment. The skin is firmer and the skin pores are tightened. The skin is enveloped with a supple, comfortable (absence of tautness) and invisible covering film.

### Examples 12 and 13:

The following two facial care compositions were prepared:

| | **12** | **13** |
|---|---|---|
| Isostearyl neopentanoate | 5 | 5 |
| Carnauba wax | 2 | 2 |
| Paraffin wax | 2 | 2 |
| Behenyl alcohol | 1.2 | 1.2 |
| Acticire | 4 | 4 |
| Polystearyl acrylate | 1 | 1 |
| Dimethicone (Xiameter PMX-200 Silicone Fluid 10cS from Dow Corning) | 5 | 5 |
| Mixture of dimethicone and dimethicone crosspolymer (Dow Corning 9041 Silicone Elastomer Blend from Dow Corning) | 5 | 5 |
| Boron nitride | 2 | 2 |
| Oxyethylenated (25 OE) cetylstearyl alcohol | 1.8 | 1.8 |
| Viscophobe DB 1000 | 2.17 | 2.17 |
| Cetyl hydroxyethylcellulose (a) | - | 0.8 |
| Pemulen TR-1 (b) | 0.4 | - |
| Glycerol | 7 | 7 |
| Triethanolamine | 0.575 | 0.175 |
| Sequestrant | 0.1 | 0.1 |
| Preserving agents | qs | qs |
| Water | qs 100% | qs 100% |
| **Hardness (kPa)** | 7.98 | 6.59 |

| | | |
|---|---|---|
| (a) Polysurf modified hydroxyethylcellulose sold by the company Ashland (b) acrylic acid/stearyl methacrylate copolymer polymerized in an ethyl acetate/cyclohexane mixture (Pemulen TR-1 Polymer from Noveon) | | |

The compositions obtained are stable after storage for two months, at room temperature (25°C) and at 45°C; they have a compact texture in accordance with their hardness.
The compositions spread easily on the skin, and are not tacky or greasy after application: they have a soft, pleasant feel, without any sensation of heaviness.
On application to facial skin, the compositions afford a comfortable (absence of tautness), supple covering on the surface of the skin, which maintains the skin at the surface, affording a sensation of containment. The skin is firmer and the skin pores are tightened.

### Comparative Examples 14 and 15 (outside the invention):

Two compositions (Examples 14, 15) outside the invention, similar to the composition of Example 3 but without the Acticire (first solid fatty substance) and without the Viscophobe DB 1000 (associative aqueous gelling agent), respectively, were prepared.

| | **3** | **14** | **15** |
|---|---|---|---|
| Isostearyl neopentanoate | 5 | 5 | 5 |
| Carnauba wax | 2 | 2 | 2 |
| Paraffin wax | 2 | 2 | 2 |
| Behenyl alcohol | 1.2 | 1.2 | 1.2 |
| **Acticire** | **4** | - | **4** |
| Polystearyl acrylate | 1 | 1 | 1 |
| Dimethicone (Xiameter PMX-200 Silicone Fluid 10cS from Dow Corning) | 5 | 5 | 5 |
| Mixture of dimethicone and dimethicone crosspolymer (Dow Corning 9041 Silicone Elastomer Blend from Dow Corning) | 5 | 5 | 5 |
| Boron nitride | 2 | 2 | 2 |
| Oxyethylenated (25 OE) cetylstearyl alcohol | 1.8 | 1.8 | 1.8 |
| Sodium polyacrylate (Cosmedia SP from Cognis) | 0.8 | 0.8 | 0.8 |
| **Viscophobe DB 1000** | **2.17** | **2.17** | - |
| Glycerol | 7 | 7 | 7 |
| Triethanolamine | 0.175 | | 0.175 |
| Sequestrant | 0.1 | 0.1 | 0.1 |
| Preserving agents | qs | qs | qs |
| Water | qs 100% | qs 100% | qs 100% |
| **Hardness (kPa) at T0** | **6.59** | **5.55** | **3.47** |
| **Hardness (kPa) at T 2 months 45°C** | **7.67** | **3.47** | **2.43** |
| **Variation of the hardness (in kPa and in %)** | **+ 1.08 (+ 16%)** | **- 2.08 (-37%)** | **-1.04 (- 30%)** |

The hardnesses of each composition were measured at T0 and also at T 2 months after storage at 45°C; the variation of the hardness of the compositions after storage was calculated.

The cosmetic properties were evaluated by applying each composition to facial skin.

By comparison with the composition of Example 3 according to the invention:
Example 14 Composition:
loss of stability: 37% drop in hardness
Application to the skin: the composition spreads less well, it is less film-forming and drags more; it imparts less of a soft feel. There is a decrease in the covering effect on the surface of the skin.
Example 15 Composition:
loss of stability: 30% drop in hardness
Application to the skin: the composition has a less thick consistency under the fingers, it is more slippery; it does not give a covering effect on the surface of the skin (this effect is not perceived).

### Example 16 (outside the invention) :

One composition outside the invention, similar to the composition of Example 3 but without the Viscophobe DB 1000 (associative aqueous gelling agent) and with in place, in same amount of active material, poly acrylamidomethyl propane sulfonic acid partially neutralized with ammonia and crosslinked (Hostacerin AMPS®)

| | |
|---|---|
| Isostearyl neopentanoate | 5% |
| Polystearyl acrylate | 1% |
| Behenyl alcohol | 1.2% |
| Carnauba wax | 2% |
| Paraffin wax | 2% |
| Dimethicone (Xiameter PMX-200 Silicone Fluid 10cS from Dow Corning) | 5% |
| Mixture of dimethicone and dimethicone crosspolymer (Dow Corning 9041 Silicone Elastomer Blend from Dow Corning) | 5% |
| Boron nitride | 2% |
| Oxyethylenated (25 OE) cetylstearyl alcohol | 1.8% |
| Acticire Methacrylic acid/methyl acrylate/ poly acrylamidomethyl propane sulfonic acid partially neutralized with | 4% |
| ammonia and crosslinked ( Hostacerin AMPS® from Clariant) | 0,5 % |
| Sodium polyacrylate (Cosmedia SP from Cognis) | 0.8% |
| Glycerol | 7% |
| Sequestrant | 0.1% |
| Preserving agents | qs |
| Water | qs 100% |

The composition obtained has a hardness of 1,74 kPa.

Comparing to the composition of the example 3, the composition of example 16 is more supple (less compact); after application on the skin, the skin is enveloped with a covering film which affording a lower sensation of containment and which have a lower matt effect. The film is also more tacky.

## Claims

1. Composition in the form of a fatty phase-in-water emulsion comprising:
(a) a first solid fatty substance with a hardness, at 25°C, ranging from 0.2 to 2 MPa;
(b) a second solid fatty substance with a hardness, at 25°C, ranging from 5 to 15 MPa;
(c) an associative aqueous gelling polymer.

2. Composition according to the preceding claim, **characterized in that** the first solid fatty substance has a melting point ranging from 28 to 45°C, preferably ranging from 28 to 40°C.

3. Composition according to either of the preceding claims, **characterized in that** the first solid fatty substance is chosen from butters of plant origin, hydrogenated plant oils, petroleum jelly, pentaerythritol ethers of C₂-C₄ polyalkylene glycol, block copolymers of ethylene oxide and/or propylene oxide and of an alkylene oxide containing from 6 to 40 carbon atoms, the copolymer having a weight-average molecular weight ranging from 5000 to 8000; esters derived from the condensation of a linear or branched, preferably saturated, C₆-C₁₀ dicarboxylic acid and of an ester of diglycerol and of optionally hydroxylated, linear or branched C₆-C₂₀ monocarboxylic acids; homopolymers of a vinyl ester containing C₈-C₃₀ alkyl groups; copolymers of allyl stearate and vinyl acetate; arachidyl propionate; saturated or unsaturated, linear or branched, optionally monohydroxylated or polyhydroxylated, C₆-C₃₀ fatty acid triglycerides; pentaerythrityl esters chosen from C₈-C₂₂ fatty acid esters of pentaerythritol, C₄-C₁₀ dicarboxylic acid esters of pentaerythritol, and mixtures thereof; esters of dilinoleyl alcohol dimer and of dilinoleic acid in which the hydroxyl groups are esterified with a mixture of phytosterols, behenyl alcohol and isostearyl alcohol; esters of dilinoleic acid and of a mixture of phytosterols, isostearyl alcohol, cetyl alcohol, stearyl alcohol and behenyl alcohol; esters of hydrogenated castor oil and of dilinoleic acid dimer; esters of hydrogenated castor oil and of C₁₆-C₂₂ fatty acids; waxes of plant origin; microcrystalline wax; vinylpyrrolidone/eicosene copolymer; and mixtures thereof.

4. Composition according to one of the preceding claims, **characterized in that** the first solid fatty substance is chosen from butters of plant origin, hydrogenated plant oils, and mixtures thereof.

5. Composition according to one of the preceding claims, **characterized in that** the first solid fatty substance is chosen from mango butter, cocoa butter; the mixture of mimosa, jojoba and sunflower plant waxes; hydrogenated jojoba oil; and mixtures thereof.

6. Composition according to one of the preceding claims, **characterized in that** the first solid fatty substance is present in a content ranging from 0.5% to 15% by weight, preferably ranging from 1% to 10% by weight and better still from 2% to 6% by weight, relative to the total weight of the composition.

7. Composition according to one of the preceding claims, **characterized in that** the second solid fatty substance has a hardness, at 25°C, ranging from 6 to 12 MPa.

8. Composition according to one of the preceding claims, **characterized in that** the second solid fatty substance has a melting point of greater than 45°C and less than or equal to 90°C.

9. Composition according to one of the preceding claims, **characterized in that** the second solid fatty substance is chosen from C12-C30 fatty acid esters of ethylene glycol, waxes of animal origin, C30-45 alkyl dimethicones, fatty alcohols comprising from 16 to 50 carbon atoms, waxes of mineral origin, waxes of plant origin, hydrogenated plant oils, polyethylene waxes, paraffin waxes, polymethylene waxes, esters of a C14-C30 fatty acid and of a plant oil, C10 to C30 alkyl (meth)acrylate homopolymers; and mixtures thereof.

10. Composition according to one of the preceding claims, **characterized in that** the second solid fatty substance is chosen from waxes of animal origin, C16-C50 fatty alcohols; waxes of mineral origin; waxes of plant origin; C10 to C30 alkyl (meth)acrylate homopolymers; and mixtures thereof.

11. Composition according to one of the preceding claims, **characterized in that** the second solid fatty substance is chosen from candelilla wax, carnauba wax; ozokerite; poly(stearyl acrylate) homopolymers, poly(behenyl acrylate) homopolymers; and mixtures thereof.

12. Composition according to one of the preceding claims, **characterized in that** the second solid fatty substance comprises a C10 to C30 alkyl (meth)acrylate homopolymer, preferably a poly(stearyl acrylate) or poly(behenyl acrylate) homopolymer, and preferentially a poly(stearyl acrylate) homopolymer; optionally combined with a wax of plant origin or a wax of mineral origin.

13. Composition according to one of the preceding claims, **characterized in that** the second solid fatty substance is present in a content ranging from 0.5% to 15% by weight, preferably ranging from 1% to 10% by weight and better still from 2% to 8% by weight, relative to the total weight of the composition.

14. Composition according to one of the preceding claims, **characterized in that** the first and second solid fatty substances are present in a total content ranging from 6% to 25% by weight, preferably ranging from 6% to 20% by weight, preferentially ranging from 8% to 16% by weight and more preferentially ranging from 10% to 14% by weight relative to the total weight of the composition.

15. Composition according to one of the preceding claims, **characterized in that** the first and second solid fatty substances are present in a mass ratio of first solid fatty substance to second solid fatty substance ranging from 0.4 to 0.8, preferably ranging from 0.45 to 0.75 and preferentially ranging from 0.5 to 0.7.

16. Composition according to one of the preceding claims, **characterized in that** the associative aqueous gelling polymer is chosen from nonionic associative polyurethane polyethers, C12-C20 fatty alcohol ethers of nonionic hydroxyethylcellulose and anionic associative acrylic polymers.

17. Composition according to one of the preceding claims, **characterized in that** the associative aqueous gelling polymer is an anionic associative acrylic polymer chosen from:
(1) copolymers derived from the polymerization of:
(i) (meth)acrylic acid,
(ii) a monomer of formula (II) below:
CH₂ = CR'CH₂OBₙR (II)
in which R' denotes H or CH₃, B denotes the ethyleneoxy group (-CH₂-CH₂-O-), n is zero or denotes an integer ranging from 1 to 100 (especially from 5 to 15) and R denotes a hydrocarbon-based group chosen from alkyl, arylalkyl, aryl, alkylaryl and cycloalkyl groups comprising from 8 to 30 carbon atoms, preferably from 10 to 24 carbon atoms and even more particularly from 16 to 20 carbon atoms.
(2) the copolymers of (i) a monomer of formula (III) below: 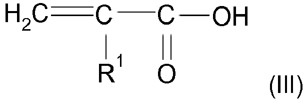 in which R¹ denotes H or CH₃ or C₂H₅,
and of (ii) a monomer of formula (IV) below:
H₂C=CR¹-COOR³ (IV)
in which R¹ denotes H or CH₃ or C₂H₅ and preferably H or CH₃, R³ denotes a C₁₀-C₃₀ and preferably C₁₂-C₂₂ alkyl group.
(3) acrylic terpolymers comprising:
(a) from 19.5% to 70% by weight of an α,β-monoethylenically unsaturated carboxylic acid containing from 3 to 5 carbon atoms,
(b) from 20% to 80% by weight of C₁-C₄ alkyl (meth)acrylates,
(c) from 0.5% to 60% by weight of a nonionic urethane macromonomer of formula (IV) below: 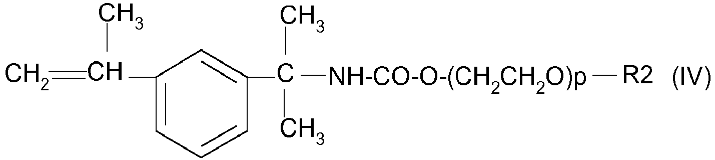 in which p ranges from 6 to 150 and R2 is chosen from linear alkyl radicals comprising from 18 to 26 and preferably from 20 to 24 carbon atoms. Preferably, the radical R2 is a behenyl radical.
(4) copolymers of an α,β-monoethylenically unsaturated carboxylic acid and of an ester of an α,β-monoethylenically unsaturated carboxylic acid and of a polyoxyethylenated C12-C30 fatty alcohol, especially with 10 to 50 ethylene oxide units, and of an ester of an α,β-monoethylenically unsaturated carboxylic acid and of a C₁-C₄ alcohol.
(5) copolymers of (meth)acrylic acid, of crosslinked C1-C4 alkyl (meth)acrylate, of polyethylene glycol C10-C30 alkyl ether methacrylate containing 25 mol of ethylene oxide and of polyethylene glycol allyl ether containing 20 ethylene oxide units/polypropylene glycol containing 5 propylene oxide units.

18. Composition according to one of the preceding claims, **characterized in that** the associative aqueous gelling polymer is a terpolymer of methacrylic acid/methyl acrylate/condensate of dimethyl meta-isopropenyl benzyl isocyanate and of polyoxyethylenated (40 OE) behenyl alcohol.

19. Composition according to one of the preceding claims, **characterized in that** the associative aqueous gelling polymer is chosen from C12-C20 fatty alcohol ethers of hydroxyethylcellulose, preferably chosen from cetyl alcohol ethers of hydroxyethylcellulose.

20. Composition according to one of the preceding claims, **characterized in that** the associative aqueous gelling polymer is a polyurethane polyether chosen from:
(1) polyurethane polyethers derived from the polycondensation of:
(i) a polyethylene glycol comprising from 120 to 250 mol of ethylene oxide,
(ii) a polyoxyethylenated monoalcohol comprising from 14 to 22 carbon atoms, comprising from 15 to 120 mol of ethylene oxide (especially from 20 to 100 OE), and
(iii) a diisocyanate containing from 6 to 20 carbon atoms, preferably chosen from methylenebis(4-cyclohexyl isocyanate) and hexamethylene diisocyanate, and preferably hexamethylene diisocyanate.
(2) polyurethane polyethers derived from the polycondensation of:
(i) a polyethylene glycol comprising from 130 to 200 mol of ethylene oxide,
(ii) a monoalcohol comprising from 8 to 22 carbon atoms, and
(iii) a diisocyanate containing from 6 to 20 carbon atoms, preferably chosen from methylenebis(4-cyclohexyl isocyanate) and hexamethylene diisocyanate, preferentially methylenebis(4-cyclohexyl isocyanate).
(3) polycondensates of polyethylene glycol containing 200 mol of ethylene oxide, ether of polyethylene glycol containing 10 mol of ethylene oxide and methylglucose, ether of polyethylene glycol containing 6 mol of ethylene oxide and of tridecyl alcohol, hexamethylene diisocyanate, and containing C16-C20 alcohol end groups.

21. Composition according to one of the preceding claims, **characterized in that** the associative aqueous gelling polymer is a polyurethane polyether chosen from:
- the polycondensate of polyethylene glycol containing 136 mol of ethylene oxide, stearyl alcohol polyoxyethylenated with 100 mol of ethylene oxide and hexamethylene diisocyanate;
- the polycondensate of polyethylene glycol containing 240 mol of ethylene oxide, polyoxyethylenated decyltetradecyl alcohol containing 20 ethylene oxide units and hexamethylene diisocyanate;
- a polycondensate of polyethylene glycol containing 150 or 180 mol of ethylene oxide, stearyl alcohol and methylenebis(4-cyclohexyl isocyanate);
- a polycondensate of polyethylene glycol containing 150 or 180 mol of ethylene oxide, decyl alcohol and methylenebis(4-cyclohexyl isocyanate);
- a polycondensate of polyethylene glycol containing 200 mol of ethylene oxide, ether of polyethylene glycol containing 10 mol of ethylene oxide and methylglucose, ether of polyethylene glycol containing 6 mol of ethylene oxide and of tridecyl alcohol, hexamethylene diisocyanate, and containing C16-C20 alcohol end groups;
and preferably chosen from:
- the polycondensate of polyethylene glycol containing 136 mol of ethylene oxide, stearyl alcohol polyoxyethylenated with 100 mol of ethylene oxide and hexamethylene diisocyanate;
- the polycondensate of polyethylene glycol containing 240 mol of ethylene oxide, polyoxyethylenated decyltetradecyl alcohol containing 20 ethylene oxide units and hexamethylene diisocyanate;
- a polycondensate of polyethylene glycol containing 150 or 180 mol of ethylene oxide, decyl alcohol and methylenebis(4-cyclohexyl isocyanate).

22. Composition according to one of the preceding claims, **characterized in that** the associative hydrophilic gelling polymer is present in a content ranging from 0.1% to 2% by weight, preferably ranging from 0.2% to 1.5% by weight and preferentially ranging from 0.2% to 1% by weight relative to the total weight of the composition.

23. Composition according to one of the preceding claims, **characterized in that** it comprises an additional aqueous gelling agent chosen from:
- crosslinked acrylic acid homopolymers, preferably crosslinked with a crosslinking agent chosen from pentaerythrityl allyl ether, sucrose allyl ether and propylene allyl ether;
- carrageenans, preferably iota-carrageenans;
- alkali metal salts of acrylic acid homopolymer, preferably sodium polyacrylate homopolymers.

24. Composition according to one of the preceding claims, **characterized in that** it comprises an additional aqueous gelling agent chosen from sodium polyacrylate homopolymers.

25. Composition according to either of Claims 23 and 24, **characterized in that** the additional aqueous gelling agent is present in the composition according to the invention in a content ranging from 0.1% to 5% by weight, preferably ranging from 0.1% to 3% by weight, preferentially ranging from 0.1% to 2% by weight and better still ranging from 0.2% to 1.5% by weight, relative to the total weight of the composition.

26. Composition according to one of the preceding claims, **characterized in that** it comprises an oil.

27. Composition according to one of the preceding claims, **characterized in that** it comprises an oil present in a content ranging from 0% to 30% by weight, preferably ranging from 0% to 20% by weight, preferentially ranging from 0% to 15% by weight and better still ranging from 5% to 15% by weight, relative to the total weight of the composition.

28. Composition according to one of the preceding claims, **characterized in that** it comprises an oil, the oil and the first and second solid fatty substances being present in a weight ratio of (first and second solid fatty substances)/oil ranging from 0.5 to 1.5, preferably ranging from 0.6 to 1.4, preferentially ranging from 0.7 to 1.3 and better still ranging from 0.9 to 1.2.

29. Composition according to one of the preceding claims, **characterized in that** it comprises a nonionic non-silicone surfactant.

30. Composition according to the preceding claim, **characterized in that** the nonionic non-silicone surfactant is chosen from:
- polyoxyethylenated C8-C30 fatty alcohols especially containing from 2 to 100 mol of ethylene oxide,
- C8-C30 fatty alcohol ethers of a sugar, in particular (C8-C30)alkyl (poly)glucosides,
- ethers of polyethylene glycol, especially containing from 20 to 120 ethylene oxide units, and of a C8-C30 fatty acid ester of glucose or of methylglucose,
- C₈-C₃₀ fatty acid esters of sorbitan,
- polyoxyethylenated C8-C30 fatty acid esters of sorbitan, especially containing from 2 to 30 mol of ethylene oxide,
- polyoxyethylenated C8-C30 fatty acid esters of sorbitan, especially containing from 2 to 100 mol of ethylene oxide,
- C8-C30 fatty acid monoesters or diesters of glycerol,
- polyglycerolated C8-C30 fatty acid esters, especially containing from 2 to 16 mol of glycerol,
- C8-C30 fatty acid esters of polyethylene glycol, especially containing from 2 to 200 ethylene oxide units,
- C8-C30 fatty acid esters of glucose or of (C1-C2)alkylglucose or of sucrose,
- and mixtures thereof;
preferably chosen from polyoxyethylenated C8-C30 fatty alcohols, especially containing from 2 to 100 mol of ethylene oxide, and (C8-C30)alkyl (poly)glucosides;
preferentially chosen from polyoxyethylenated C8-C30 fatty alcohols especially containing from 2 to 100 mol of ethylene oxide.

31. Composition according to Claim 29 or 30, **characterized in that** the nonionic non-silicone surfactant is present in the composition according to the invention in a content ranging from 0.1% to 4% by weight, preferably from 0.1% to 3% by weight, better still from 0.4% to 2.5% by weight and even better still ranging from 0.4% to 2% by weight, relative to the total weight of the composition.

32. Composition according to one of the preceding claims, **characterized in that** it comprises water in a content ranging from 25% to 90% by weight, preferably from 30% to 80% by weight and better still from 40% to 70% by weight, relative to the total weight of the composition.

33. Composition according to one of the preceding claims, **characterized in that** it comprises a non-emulsifying elastomeric organopolysiloxane.

34. Composition according to one of the preceding claims, **characterized in that** it has a hardness, at 25°C, ranging from 1 to 30 kPa, preferably ranging from 1 to 15 kPa and preferentially ranging from 1 to 10 kPa.

35. Process, especially a cosmetic process, for treating the skin, comprising the application to the skin of a composition according to one of the preceding claims.

36. Process according to the preceding claim, **characterized in that** the process is intended for treating slackening of the skin and/or for reducing the appearance of the fine lines of the skin.

## Patentansprüche

1. Zusammensetzung in Form einer Fettphase-in-Wasser-Emulsion, umfassend:
(a) eine erste feste Fettsubstanz mit einer Härte bei 25 °C im Bereich vaon 0,2 bis 2 MPa;
(b) eine zweite feste Fettsubstanz mit einer Härte bei 25 °C im Bereich von 5 bis 15 MPa;
(c) ein assoziatives wässriges gelierendes Polymer.

2. Zusammensetzung nach dem vorhergehenden Anspruch, **dadurch gekennzeichnet, dass** die erste feste Fettsubstanz einen Schmelzpunkt im Bereich von 28 bis 45 °C, vorzugsweise im Bereich von 28 bis 40 °C, aufweist.

3. Zusammensetzung nach einem der vorhergehenden Ansprüche, **dadurch gekennzeichnet, dass** die erste feste Fettsubstanz aus Buttern pflanzlicher Herkunft, hydrierten Pflanzenölen, Vaseline, Pentaerythritolestern von C₂-C₄-Polyalkylenglykol, Blockcopolymeren von Ethylenoxid und/oder Propylenoxid und einem Alkylenoxid mit 6 bis 40 Kohlenstoffatomen, wobei das Copolymer ein gewichtsmittleres Molekulargewicht im Bereich von 5000 bis 8000 aufweist; Estern aus der Kondensation einer linearen oder verzweigten, vorzugsweise gesättigten, C₆-C₁₀-Dicarbonsäure und einem Ester von Diglycerin und gegebenenfalls hydroxylierten, linearen oder verzweigten C₆-C₂₀-Monocarbonsäuren; Homopolymeren eines Vinylesters mit C₈-C₃₀-Alkylgruppen; Copolymeren von Allylstearat und Vinylacetat; Arachidylpropionat; gesättigten oder ungesättigten, linearen oder verzweigten, gegebenenfalls monohydroxylierten oder polyhydroxylierten C₆-C₃₀-Fettsäuretriglyceriden; Pentaerythritylestern, die aus C₈-C₂₂-Fettsäureestern von Pentaerythritol, C₄-C₁₀-Dicarbonsäureestern von Pentaerythritol und Mischungen davon ausgewählt sind; Estern von Dilinoleylalkoholdimer und Dilinolsäure, in denen die Hydroxylgruppen mit einer Mischung von Phytostyrolen, Behenylalkohol und Isostearylalkohol verestert sind; Estern von Dilinolsäure und einer Mischung von Phytosterolen, Isostearylalkohol, Cetylalkohol, Stearylalkohol und Behenylalkohol; Estern von hydriertem Rizinusöl und Dilinolsäuredimer; Estern von hydriertem Rizinusöl und C₁₆-C₂₂-Fettsäuren; Wachsen pflanzlicher Herkunft; mikrokristallinem Wachs; Vinylpyrrolidon/Eicosen und Mischungen davon ausgewählt ist.

4. Zusammensetzung nach einem der vorhergehenden Ansprüche, **dadurch gekennzeichnet, dass** die erste feste Fettsubstanz aus Buttern pflanzlicher Herkunft, hydrierten Pflanzenölen und Mischungen davon ausgewählt ist.

5. Zusammensetzung nach einem der vorhergehenden Ansprüche, **dadurch gekennzeichnet, dass** die erste feste Fettsubstanz aus Mangobutter, Kakaobutter; der Mischung von Mimosen-, Jojoba- und Sonnenblumenpflanzenwachsen; hydriertem Jojobaöl und Mischungen davon ausgewählt ist.

6. Zusammensetzung nach einem der vorhergehenden Ansprüche, **dadurch gekennzeichnet, dass** die erste feste Fettsubstanz in einem Gehalt im Bereich von 0,5 bis 15 Gew.-%, vorzugsweise im Bereich von 1 bis 10 Gew.-% und noch besser von 2 bis 6 Gew.-%, bezogen auf das Gesamtgewicht der Zusammensetzung, vorliegt.

7. Zusammensetzung nach einem der vorhergehenden Ansprüche, **dadurch gekennzeichnet, dass** die zweite feste Fettsubstanz eine Härte bei 25 °C im Bereich von 6 bis 12 MPa aufweist.

8. Zusammensetzung nach einem der vorhergehenden Ansprüche, **dadurch gekennzeichnet, dass** die zweite feste Fettsubstanz einen Schmelzpunkt von mehr als 45 °C und 90 °C oder weniger aufweist.

9. Zusammensetzung nach einem der vorhergehenden Ansprüche, **dadurch gekennzeichnet, dass** die zweite feste Fettsubstanz aus C₁₂-C₃₀-Fettsäureestern von Ethylenglykol, Wachsen tierischer Herkunft, C₃₀₋₄₅-Alkyldimethiconen, Fettalkoholen mit 16 bis 50 Kohlenstoffatomen, Wachsen mineralischer Herkunft, Wachsen pflanzlicher Herkunft, hydrierten Pflanzenölen, Polyethylenwachsen, Paraffinwachsen, Polymethylenwachsen, Estern einer C₁₄-C₃₀-Fettsäure und eines Pflanzenöls, C₁₀- bis C₃₀-Alkyl (meth) acrylat-Homopolymeren und Mischungen davon ausgewählt ist.

10. Zusammensetzung nach einem der vorhergehenden Ansprüche, **dadurch gekennzeichnet, dass** die zweite feste Fettsubstanz aus Wachsen tierischer Herkunft, C₁₆-C₅₀-Fettalkoholen; Wachsen mineralischer Herkunft; Wachsen pflanzlicher Herkunft; C₁₀- bis C₃₀-Alkyl (meth)-acrylat-Homopolymeren und Mischungen davon ausgewählt ist.

11. Zusammensetzung nach einem der vorhergehenden Ansprüche, **dadurch gekennzeichnet, dass** die zweite feste Fettsubstanz aus Candelillawachs, Carnaubawachs; Ozokerit; Poly(stearylacrylat)-Homopolymeren, Poly(behenylacrylat)-Homopolymeren und Mischungen davon ausgewählt ist.

12. Zusammensetzung nach einem der vorhergehenden Ansprüche, **dadurch gekennzeichnet, dass** die zweite feste Fettsubstanz ein C₁₀- bis C₃₀-Alkyl (meth) acrylat-Homopolymer, vorzugsweise ein Poly(stearylacrylat)-oder Poly(behenylacrylat)-Homopolymer und bevorzugt ein Poly(stearylacrylat)-Homopolymer; gegebenenfalls in Kombination mit einem Wachs pflanzlicher Herkunft oder einem Wachs mineralischer Herkunft, umfasst.

13. Zusammensetzung nach einem der vorhergehenden Ansprüche, **dadurch gekennzeichnet, dass** die zweite feste Fettsubstanz in einem Gehalt im Bereich von 0,5 bis 15 Gew.-%, vorzugsweise im Bereich von 1 bis 10 Gew.-% und noch besser von 2 bis 8 Gew.-%, bezogen auf das Gesamtgewicht der Zusammensetzung, vorliegt.

14. Zusammensetzung nach einem der vorhergehenden Ansprüche, **dadurch gekennzeichnet, dass** die erste feste Fettsubstanz und die zweite feste Fettsubstanz in einer Gesamtmenge im Bereich von 6 bis 25 Gew.-%, vorzugsweise im Bereich von 6 bis 20 Gew.-%, bevorzugt im Bereich von 8 bis 16 Gew.-% und weiter bevorzugt im Bereich von 10 bis 14 Gew.-%, bezogen auf das Gesamtgewicht der Zusammensetzung, vorliegen.

15. Zusammensetzung nach einem der vorhergehenden Ansprüche, **dadurch gekennzeichnet, dass** die erste feste Fettsubstanz und die zweite feste Fettsubstanz in einem Massenverhältnis von erster fester Fettsubstanz zu zweiter fester Fettsubstanz im Bereich von 0,4 bis 0,8, vorzugsweise im Bereich von 0,45 bis 0,75 und bevorzugt im Bereich von 0,5 bis 0,7 vorliegen.

16. Zusammensetzung nach einem der vorhergehenden Ansprüche, **dadurch gekennzeichnet, dass** das assoziative wässrige gelierende Polymer aus nichtionischen assoziativen Polyurethanpolyethern, C₁₂-C₂₀-Fettalkoholethern von nichtionischer Hydroxyethylcellulose und anionischen assoziativen Acrylpolymeren ausgewählt ist.

17. Zusammensetzung nach einem der vorhergehenden Ansprüche, **dadurch gekennzeichnet, dass** es sich bei dem assoziativen wässrigen gelierenden Polymer um ein anionisches assoziatives Acrylpolymer handelt, das ausgewählt ist aus:
(1) Copolymeren aus der Polymerisation von:
(i) (Meth)acrylsäure,
(ii) einem Monomer der nachstehenden Formel (II):
CH₂ = CR'CH₂OBₙR (II),
in der R' für H oder CH₃ steht, B für die Ethylenoxygruppe (-CH₂-CH₂-O-) steht, n null ist oder für eine ganze Zahl im Bereich von 1 bis 100 (insbesondere von 5 bis 15) steht und R für eine Gruppe auf Kohlenwasserstoffbasis, die aus Alkyl-, Arylalkyl-, Aryl-, Alkylaryl- und Cycloalkylgruppen mit 8 bis 30 Kohlenstoffatomen, vorzugsweise 10 bis 24 Kohlenstoffatomen und noch spezieller 16 bis 20 Kohlenstoffatomen ausgewählt ist, steht;
(2) den Copolymeren von (i) einem Monomer der nachstehenden Formel (III): 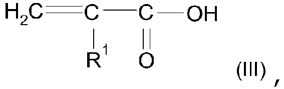 in der R¹ für H oder CH₃ oder C₂H₅ steht,
und (ii) einem Monomer der nachstehenden Formel (IV) :
H₂C=CR¹-COOR³ (IV),
in der R¹ für H oder CH₃ oder C₂H₅ und vorzugsweise H oder CH₃ steht, R³ für eine C₁₀-C₃₀- und vorzugsweise C₁₂-C₂₂-Alkylgruppe steht;
(3) Acrylterpolymeren, umfassend:
(a) 19,5 bis 70 Gew.-% einer α,β-monoethylenisch ungesättigten Carbonsäure mit 3 bis 5 Kohlenstoffatomen,
(b) 20 bis 80 Gew.-% Alkyl(meth)acrylate,
(c) 0,5 bis 60 Gew.-% eines nichtionischen Urethan-Makromonomers der nachstehenden Formel (IV): 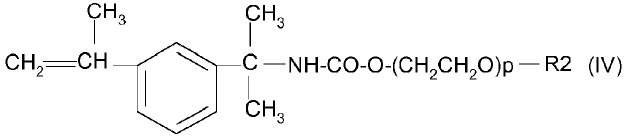 in der p im Bereich von 6 bis 150 liegt und R2 aus linearen Alkylresten mit 18 bis 26 und vorzugsweise 20 bis 24 Kohlenstoffatomen ausgewählt ist; vorzugsweise der Rest R2 ein Behenylrest ist;
(4) Copolymeren einer α,β-monoethylenisch ungesättigten Carbonsäure und eines Esters einer α,β-monoethylenisch ungesättigten Carbonsäure und eines polyoxyethylenierten C₁₂-C₃₀-Fettalkohols, insbesondere mit 10 bis 50 Ethylenoxid-Einheiten, und eines Esters einer α,β-monoethylenisch ungesättigten Carbonsäure und eines C₁-C₄-Alkohols;
(5) Copolymeren von (Meth)acrylsäure, vernetztem C₁-C₄-Alkyl (meth) acrylat, Polyethylenglykol-C₁₀-C₃₀-Alkylethermethacrylat mit 25 mol Ethylenoxid und Polyethylenglykolallylether mit 20 Ethylenoxid-Einheiten/Polypropylenglykol mit 5 Propylenoxid-Einheiten.

18. Zusammensetzung nach einem der vorhergehenden Ansprüche, **dadurch gekennzeichnet, dass** es sich bei dem assoziativen wässrigen gelierenden Polymer um ein Terpolymer von Methacrylsäure/Methylacrylat/Kondensat von Dimethyl-meta-isopropenylbenzylisocyanat und polyoxyethyleniertem (40 OE) Behenylalkohol handelt.

19. Zusammensetzung nach einem der vorhergehenden Ansprüche, **dadurch gekennzeichnet, dass** das assoziative wässrige gelierende Polymer aus C₁₂-C₂₀-Fettalkoholethern von Hydroxyethylcellulose, vorzugsweise aus Cetylalkoholethern von Hydroxyethylcellulose, ausgewählt ist.

20. Zusammensetzung nach einem der vorhergehenden Ansprüche, **dadurch gekennzeichnet, dass** es sich bei dem assoziativen wässrigen gelierenden Polymer um einen Polyurethanpolyether handelt, der ausgewählt ist aus:
(1) Polyurethanpolyethern aus der Polykondensation von:
(i) einem Polyethylenglykol mit 120 bis 250 mol Ethylenoxid,
(ii) einem polyoxyethylenierten Monoalkohol mit 14 bis 22 Kohlenstoffatomen und 15 bis 120 mol Ethylenoxid (insbesondere 20 bis 100 OE) und
(iii) einem Diisocyanat mit 6 bis 20 Kohlenstoffatomen, vorzugsweise ausgewählt aus Methylenbis(4-cyclohexylisocyanat) und Hexamethylendiisocyanat, und vorzugsweise Hexamethylendiisocyanat;
(2) Polyurethanpolyethern aus der Polykondensation von:
(i) einem Polyethylenglykol mit 130 bis 200 mol Ethylenoxid,
(ii) einem Monoalkohol mit 8 bis 22 Kohlenstoffatomen und
(iii) einem Diisocyanat mit 6 bis 20 Kohlenstoffatomen, vorzugsweise ausgewählt aus Methylenbis(4-cyclohexylisocyanat) und Hexamethylendiisocyanat, und vorzugsweise Methylenbis(4-cyclohexylisocyanat);
(3) Polykondensaten von Polyethylenglykol mit 200 mol Ethylenoxid, Ether von Polyethylenglykol mit 10 mol Ethylenoxid und Methylglucose, Ether von Polyethylenglykol mit 6 mol Ethylenoxid und Tridecylalkohol, Hexamethylendiisocyanat und mit C₁₆-C₂₀-Alkohol-Endgruppen.

21. Zusammensetzung nach einem der vorhergehenden Ansprüche, **dadurch gekennzeichnet, dass** es sich bei dem assoziativen wässrigen gelierenden Polymer um einen Polyurethanpolyether handelt, der ausgewählt ist aus:
- dem Polykondensat von Polyethylenglykol mit 136 mol Ethylenoxid, mit 100 mol Ethylenoxid polyoxyethyleniertem Stearylalkohol und Hexamethylendiisocyanat;
- dem Polykondensat von Polyethylenglykol mit 240 mol Ethylenoxid, polyoxyethyleniertem Decyltetradecylalkohol mit 20 Ethylenoxid-Einheiten und Hexamethylendiisocyanat;
- einem Polykondensat von Polyethylenglykol mit 150 oder 180 mol Ethylenoxid, Stearylalkohol und Methylenbis(4-cyclohexylisocyanat) ;
- einem Polykondensat von Polyethylenglykol mit 150 oder 180 mol Ethylenoxid, Decylalkohol und Methylenbis(4-cyclohexylisocyanat) ;
- einem Polykondensat von Polyethylenglykol mit 200 mol Ethylenoxid, Ether von Polyethylenglykol mit 10 mol Ethylenoxid und Methylglucose, Ether von Polyethylenglykol mit 6 mol Ethylenoxid und Tridecylalkohol, Hexamethylendiisocyanat und mit C₁₆-C₂₀-Alkohol-Endgruppen;
und vorzugsweise ausgewählt ist aus:
- dem Polykondensat von Polyethylenglykol mit 136 mol Ethylenoxid, mit 100 mol Ethylenoxid polyoxyethyleniertem Stearylalkohol und Hexamethylendiisocyanat;
- dem Polykondensat von Polyethylenglykol mit 240 mol Ethylenoxid, polyoxyethyleniertem Decyltetradecylalkohol mit 20 Ethylenoxid-Einheiten und Hexamethylendiisocyanat;
- einem Polykondensat von Polyethylenglykol mit 150 oder 180 mol Ethylenoxid, Decylalkohol und Methylenbis(4-cyclohexylisocyanat).

22. Zusammensetzung nach einem der vorhergehenden Ansprüche, **dadurch gekennzeichnet, dass** das assoziative hydrophile gelierende Polymer in einem Gehalt im Bereich von 0,1 bis 2 Gew.-%, vorzugsweise im Bereich von 0,2 bis 1,5 Gew.-% und bevorzugt im Bereich von 0,2 bis 1 Gew.-%, bezogen auf das Gesamtgewicht der Zusammensetzung, vorliegt.

23. Zusammensetzung nach einem der vorhergehenden Ansprüche, **dadurch gekennzeichnet, dass** sie ein zusätzliches wässriges Geliermittel umfasst, das ausgewählt ist aus:
- vernetzten Acrylsäure-Homopolymeren, die vorzugsweise mit einem Vernetzungsmittel, das aus Pentaerythritylallylether, Saccharoseallylether und Propylenallylether ausgewählt ist, vernetzt sind;
- Carrageenanen, vorzugsweise iota-Carrageenanen;
- Alkalimetallsalzen von Acrylsäure-Homopolymer, vorzugsweise Natriumpolyacrylat-Homopolymeren.

24. Zusammensetzung nach einem der vorhergehenden Ansprüche, **dadurch gekennzeichnet, dass** es ein zusätzliches wässriges Geliermittel umfasst, das aus Natriumpolyacrylat-Homopolymeren ausgewählt ist.

25. Zusammensetzung nach Anspruch 23 oder 24, **dadurch gekennzeichnet, dass** das zusätzliche wässrige Geliermittel in der erfindungsgemäßen Zusammensetzung in einem Gehalt im Bereich von 0,1 bis 5 Gew.-%, vorzugsweise im Bereich von 0,1 bis 3 Gew.-%, bevorzugt im Bereich von 0,1 bis 2 Gew.-% und noch besser im Bereich von 0,2 bis 1,5 Gew.-%, bezogen auf das Gesamtgewicht der Zusammensetzung, vorliegt.

26. Zusammensetzung nach einem der vorhergehenden Ansprüche, **dadurch gekennzeichnet, dass** sie ein Öl umfasst.

27. Zusammensetzung nach einem der vorhergehenden Ansprüche, **dadurch gekennzeichnet, dass** sie ein Öl umfasst, das in einem Gehalt im Bereich von 0 bis 30 Gew.-%, vorzugsweise im Bereich von 0 bis 20 Gew.-%, bevorzugt im Bereich von 0 bis 15 Gew.-% und noch besser im Bereich von 5 bis 15 Gew.-%, bezogen auf das Gesamtgewicht der Zusammensetzung, vorliegt.

28. Zusammensetzung nach einem der vorhergehenden Ansprüche, **dadurch gekennzeichnet, dass** sie ein Öl umfasst, wobei das Öl und die erste feste Fettsubstanz und die zweite feste Fettsubstanz in einem Gewichtsverhältnis von (erste feste Fettsubstanz und zweite feste Fettsubstanz)/Öl im Bereich von 0,5 bis 1,5, vorzugsweise im Bereich von 0,6 bis 1,4, bevorzugt im Bereich von 0,7 bis 1,3 und noch besser im Bereich von 0,9 bis 1,2 vorliegen.

29. Zusammensetzung nach einem der vorhergehenden Ansprüche, **dadurch gekennzeichnet, dass** sie ein nichtionisches Nichtsilikontensid umfasst.

30. Zusammensetzung nach dem vorhergehenden Anspruch, **dadurch gekennzeichnet, dass** das nichtionische Nichtsilikontensid ausgewählt ist aus:
- polyoxyethylenierten C₈-C₃₀-Fettalkoholen, insbesondere mit 2 bis 100 mol Ethylenoxid,
- C₈-C₃₀-Fettalkoholethern eines Zuckers, insbesondere (C₈-C₃₀)-Alykl(poly)glucosiden,
- Ethern von Polyethylenglykol, insbesondere mit 20 bis 120 Ethylenoxid-Einheiten, und eines C₈-C₃₀-Fettsäureesters von Glucose oder Methylglucose,
- C₈-C₃₀-Fettsäureestern von Sorbitan,
- polyoxyethylenierten C₈-C₃₀-Fettsäureestern von Sorbitan, insbesondere mit 2 bis 30 mol Ethylenoxid,
- polyoxyethylenierten C₈-C₃₀-Fettsäureestern von Sorbitan, insbesondere mit 2 bis 100 mol Ethylenoxid,
- C₈-C₃₀-Fettsäuremonoestern oder -diestern von Glycerin,
- polyglycerinierten C₈-C₃₀-Fettsäureestern, insbesondere mit 2 bis 16 mol Glycerin,
- C₈-C₃₀-Fettsäureestern von Polyethylenglykol, insbesondere mit 2 bis 200 Ethylenoxid-Einheiten,
- C₈-C₃₀-Fettsäureestern von Glucose oder von (C₁-C₂)-Alkylglucose oder von Saccharose
und Mischungen davon;
vorzugsweise ausgewählt aus polyoxyethylenierten C₈-C₃₀-Fettalkoholen, insbesondere mit 2 bis 100 mol Ethylenoxid, und (C₈-C₃₀)-Alkyl(poly)glucosiden;
bevorzugt ausgewählt aus polyoxyethylenierten C₈-C₃₀-Fettalkoholen, insbesondere mit 2 bis 100 mol Ethylenoxid.

31. Zusammensetzung nach Anspruch 29 oder 30, **dadurch gekennzeichnet, dass** das nichtionische Nichtsilikontensid in der erfindungsgemäßen Zusammensetzung in einem Gehalt im Bereich von 0,1 bis 4 Gew.-%, vorzugsweise von 0,1 bis 3 Gew.-%, noch besser von 0,4 bis 2,5 Gew.-% und sogar noch besser im Bereich von 0,4 bis 2 Gew.-%, bezogen auf das Gesamtgewicht der Zusammensetzung, vorliegt.

32. Zusammensetzung nach einem der vorhergehenden Ansprüche, **dadurch gekennzeichnet, dass** sie Wasser in einem Gehalt von 25 bis 90 Gew.-%, vorzugsweise von 30 bis 80 Gew.-% und noch besser von 40 bis 70 Gew.-%, bezogen auf das Gesamtgewicht der Zusammensetzung, umfasst.

33. Zusammensetzung nach einem der vorhergehenden Ansprüche, **dadurch gekennzeichnet, dass** sie ein nichtemulgierendes elastomeres Organopolysiloxan umfasst.

34. Zusammensetzung nach einem der vorhergehenden Ansprüche, **dadurch gekennzeichnet, dass** sie eine Härte bei 25 °C im Bereich von 1 bis 30 kPa, vorzugsweise im Bereich von 1 bis 15 kPa und bevorzugt im Bereich von 1 bis 10 kPa aufweist.

35. Verfahren, insbesondere kosmetisches Verfahren, zur Behandlung der Haut, bei dem man auf die Haut eine Zusammensetzung nach einem der vorhergehenden Ansprüche aufbringt.

36. Verfahren nach dem vorhergehenden Anspruch, **dadurch gekennzeichnet, dass** das Verfahren zur Behandlung einer Erschlaffung der Haut und/oder zur Verringerung des Erscheinens der feinen Linien der Haut vorgesehen ist.

## Revendications

1. Composition sous la forme d'une émulsion phase grasse-dans-eau comprenant :
(a) une première substance grasse solide dotée d'une dureté, à 25 °C, allant de 0,2 à 2 MPa ;
(b) une deuxième substance grasse solide dotée d'une dureté, à 25 °C, allant de 5 à 15 MPa ;
(c) un polymère gélifiant aqueux associatif.

2. Composition selon la revendication précédente, **caractérisée** en ce la première substance grasse solide a un point de fusion allant de 28 à 45 °C, de préférence allant de 28 à 40 °C.

3. Composition selon l'une ou l'autre des revendications précédentes, **caractérisée en ce que** la première substance grasse solide est choisie parmi des beurres d'origine végétale, des huiles végétales hydrogénées, la gelée de pétrole, des éthers de pentaérythritol de polyalkylène glycol en C₂-C₄, des copolymères à blocs ou d'oxyde d'éthylène et/ou d'oxyde de propylène et d'un oxyde d'alkylène possédant de 6 à 40 atomes de carbones, le copolymère possédant un poids moléculaire moyen en poids allant de 5 000 à 8 000 ; des esters issus de la condensation d'un acide dicarboxylique linéaire ou ramifié, de préférence saturé, en C₆-C₁₀ et d'un ester de diglycérol et d'acides monocarboxyliques linéaires ou ramifiés, éventuellement hydroxylés, en C₆-C₂₀ ; des homopolymères d'un ester de vinyle contenant des groupes alkyle en C₈-C₃₀ ; des copolymères de stéarate d'allyle et d'acétate de vinyle ; le propionate d'arachidyle ; des triglycérides d'acide gras, saturé ou non, linéaire ou ramifié, éventuellement monohydroxylé ou polyhydroxylé, en C₆-C₃₀ ; des esters de pentaérythritol choisis parmi des esters de pentaérythritol d'acide gras en C₈-C₂₂, des esters de pentaérythritol d'acide dicarboxylique en C₄-C₁₀, et des mélanges de ceux-ci ; des esters de dimère d'alcool dilinoléique et de d'acide dilinoléique, dans laquelle les groupes hydroxyles sont estérifiés avec un mélange de phytostérols, d'alcool béhénylique et d'alcool isostéarique ; des esters d'acide dilinoléique et d'un mélange de phytostérols, d'alcool isostéarique, d'alcool cétylique, d'alcool stéarylique et d'alcool béhénylique ; des esters d'huile de ricin hydrogénée et du dimère d'acide dilinoléique ; des esters d'huile de ricin hydrogénée et d'acides gras en C₁₆-C₂₂ ; des cires d'origine végétale ; une cire microcristalline ; un copolymère de vinylpyrrolidone/éicosène ; et des mélanges de ceux-ci.

4. Composition selon l'une des revendications précédentes, **caractérisée en ce que** la première substance grasse solide est choisie parmi des beurres d'origine végétale, des huiles végétales hydrogénées, et des mélanges de ceux-ci.

5. Composition selon l'une des revendications précédentes, **caractérisée en ce que** la première substance grasse solide est choisie parmi le beurre de mangue, le beurre de cacao ; le mélange de cires végétales de mimosa, de jojoba et de tournesol ; l'huile de jojoba hydrogénée ; et des mélanges de ceux-ci.

6. Composition selon l'une des revendications précédentes, **caractérisée en ce que** la première substance grasse solide est présente en une teneur allant de 0,5 % à 15 % en poids, de préférence allant de 1 % à 10 % en poids et encore mieux de 2 % à 6 % en poids, par rapport au poids total de la composition.

7. Composition selon l'une des revendications précédentes, **caractérisée en ce que** la deuxième substance grasse solide possède une dureté, à 25 °C, allant de 6 à 12 MPa.

8. Composition selon l'une des revendications précédentes, **caractérisée en ce que** la deuxième substance grasse solide possède un point de fusion supérieur à 45 °C et inférieur ou égal à 90 °C.

9. Composition selon l'une des revendications précédentes, **caractérisée en ce que** la deuxième substance grasse solide est choisie parmi des esters d'éthylèneglycol d'acide gras en C₁₂-C₃₀, des cires d'origine animale, des C₃₀₋₄₅ alkyl-diméthicones, des alcools gras comprenant de 16 à 50 atomes de carbone, des cires d'origine minérale, des cires d'origine végétale, des huiles végétales hydrogénées, des cires de polyéthylène, des cires de paraffine, des cires de polyméthylène, des esters d'acide gras en C₁₄-C₃₀ d'une huile végétale, des homopolymères de (méth)acrylate d'alkyle en C₁₀ à C₃₀ ; et des mélanges de ceux-ci.

10. Composition selon l'une des revendications précédentes, **caractérisée en ce que** la deuxième substance grasse solide est choisie parmi les cires d'origine animale, les alcools gras en C₁₆-C₅₀ ; les cires d'origine minérale; les cires d'origine végétale ; les homopolymères de (méth)acrylate d'alkyle en C₁₀ à C₃₀ ; et des mélanges de ceux-ci.

11. Composition selon l'une des revendications précédentes, **caractérisée en ce que** la deuxième substance grasse solide est choisie parmi la cire de candelilla, la cire de carnauba ; l'ozokérite ; les homopolymères de poly(acrylate de stéaryle), les homopolymères de poly(acrylate de béhényle) ; et des mélanges de ceux-ci.

12. Composition selon l'une des revendications précédentes, **caractérisée en ce que** la deuxième substance grasse solide comprend un homopolymère de (méth)acrylate d'alkyle en C₁₀ à C₃₀, de préférence un homopolymère de poly(acrylate de stéaryle) ou de poly(acrylate de béhényle), et préférentiellement un homopolymère de poly(acrylate de stéaryle) ; éventuellement combinée avec une cire d'origine végétale ou une cire d'origine minérale.

13. Composition selon l'une des revendications précédentes, **caractérisée en ce que** la deuxième substance grasse solide est présente en une teneur allant de 0,5 % à 15 % en poids, de préférence allant de 1 % à 10 % en poids, et encore mieux de 2 % à 8 % en poids, par rapport au poids total de la composition.

14. Composition selon l'une des revendications précédentes, **caractérisée en ce que** les première et deuxième substances grasses solides sont présentes en une teneur totale allant de 6 % à 25 % en poids, de préférence allant de 6 % à 20 % en poids, préférentiellement allant de 8 % à 16 % en poids, et plus préférentiellement allant de 10 % à 14 % en poids, par rapport au poids total de la composition.

15. Composition selon l'une des revendications précédentes, **caractérisée en ce que** les première et deuxième substances grasses solides sont présentes selon un rapport massique de première substance grasse solide sur deuxième substance grasse solide allant de 0,4 à 0,8, de préférence allant de 0,45 à 0,75, et préférentiellement allant de 0,5 à 0,7.

16. Composition selon l'une des revendications précédentes, **caractérisée en ce que** le polymère gélifiant aqueux associatif est choisi parmi les polyuréthane polyéthers associatifs non ioniques, les éthers d'alcool gras en C₁₂-C₂₀ et d'hydroxyéthylcellulose non ionique et les polymères acryliques associatifs anioniques.

17. Composition selon l'une des revendications précédentes, **caractérisée en ce que** le polymère gélifiant aqueux associatif est un polymère acrylique associatif anionique choisi parmi :
(1) les copolymères issus de la polymérisation :
(i) d'acide (méth)acrylique,
(ii) d'un monomère de formule (II) ci-dessous :
CH₂=CR'CH₂OBₙR (II)
dans laquelle R' désigne H ou CH₃, B désigne le groupe éthylèneoxy (-CH₂-CH₂-O-), n est zéro ou désigne un entier allant de 1 à 100 (notamment de 5 à 15), et R désigne un groupe à base d'hydrocarbure choisi parmi les groupes alkyle, arylalkyle, aryle, alkylaryle et cycloalkyle, comprenant de 8 à 30 atomes de carbone, de préférence 10 à 24 atomes de carbone, et plus particulièrement encore de 16 à 20 atomes de carbone.
(2) les copolymères de (i) monomère de formule (III) ci-dessous : 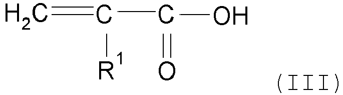 dans laquelle R¹ désigne H ou CH₃ ou C₂H₅,
et (ii) d'un monomère de formule (IV) ci-dessous :
H₂C=CR¹-COOR³ (IV)
dans laquelle R¹ désigne H ou CH₃ ou C₂H₅, et de préférence H ou CH₃, R³ désigne un groupe alkyle en C₁₀-C₃₀, et de préférence en C₁₂-C₂₂ ;
(3) les terpolymères acryliques comprenant :
(a) de 19,5 % à 70 % en poids d'un acide carboxylique monoéthyléniquement α,β-insaturé contenant de 3 à 5 atomes de carbone,
(b) de 20 % à 80 % en poids de (méth)acrylates d'alkyle en C₁-C₄,
(c) de 0,5 % à 60 % en poids d'un macromonomère d'uréthane non-ionique de formule (IV) ci-dessous : 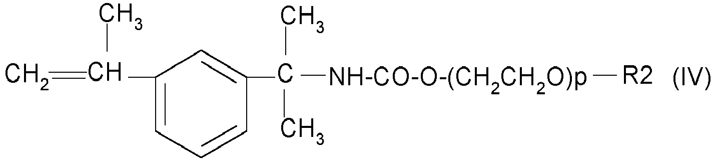 dans laquelle p va de 6 à 150 et R2 est choisi parmi les radicaux alkyle linéaires comprenant de 18 à 26 et de préférence de 20 à 24 atomes de carbone. De préférence, le radical R2 est un radical béhényle.
(4) les copolymères d'un acide carboxylique monoéthyléniquement α,β-insaturé et d'un ester d'acide carboxylique monoéthyléniquement α,β-insaturé et d'un alcool gras en C12-C30 polyoxyéthylénisé, notamment comportant 10 à 50 motifs d'oxyde d'éthylène, et d'un ester d'acide carboxylique monoéthyléniquement α,β-insaturé et d'un alcool en C₁-C₄.
(5) les copolymères d'acide (méth)acrylique, de (méth)acrylate d'alkyle en C1-C4 réticulé, de méthacrylate d'éther de C10-C30 alkyle de polyéthylèneglycol contenant 25 moles d'oxyde d'éthylène et d'éther d'allyle de polyéthylèneglycol contenant 20 motifs d'oxyde d'éthylène / polypropylèneglycol contenant 5 motifs d'oxyde de propylène.

18. Composition selon l'une des revendications précédentes, **caractérisée en ce que** le polymère gélifiant aqueux associatif est un terpolymère d'acide méthacrylique/ acrylate de méthyle/ condensat de diméthyl-méta-isopropénylbenzylisocyanate et d'alcool béhénylique polyoxyéthylénisé (40 OE).

19. Composition selon l'une des revendications précédentes, **caractérisée en ce que** le polymère gélifiant aqueux associatif est choisi parmi les éthers d'alcool gras en C12-C20 d'hydroxyéthylcellulose, de préférence choisi parmi les éthers d'alcool cétylique d'hydroxyéthylcellulose.

20. Composition selon l'une des revendications précédentes, **caractérisée en ce que** le polymère gélifiant aqueux associatif est un polyuréthane polyéther choisi parmi :
(1) les polyuréthane polyéthers issus de la polycondensation de :
(i) un polyéthylèneglycol comprenant de 120 à 250 moles d'oxyde d'éthylène,
(ii) un monoalcool polyoxyéthylénisé comprenant de 14 à 22 atomes de carbone, comprenant de 15 à 120 moles d'oxyde d'éthylène (notamment de 20 à 100 OE), et
(iii) un diisocyanate contenant de 6 à 20 atomes de carbone, de préférence choisi parmi le méthylène-bis(4-cyclohexylisocyanate) et le diisocyanate d'hexamethylène, et préférentiellement le diisocyanate d'hexamethylène.
(2) les polyuréthane polyéthers issus de la polycondensation de :
(i) un polyéthylèneglycol comprenant de 130 à 200 moles d'oxyde d'éthylène,
(ii) un monoalcool comprenant de 8 à 22 atomes de carbone, et
(iii) un diisocyanate ayant de 6 à 20 atomes de carbone, de préférence choisi parmi le méthylène-bis(4-cyclohexylisocyanate) et le diisocyanate d'hexamethylène, préférentiellement le méthylène-bis (4-cyclohexylisocyanate).
(3) les polycondensats de polyéthylèneglycol contenant 200 moles d'oxyde d'éthylène, d'éther de polyéthylèneglycol contenant 10 moles d'oxyde d'éthylène et de méthylglucose, d'éther de polyéthylèneglycol contenant 6 moles d'oxyde d'éthylène et d'alcool tridécylique, de diisocyanate d'hexamethylène, et contenant des groupes terminaux de type alcool en C16-C20.

21. Composition selon l'une des revendications précédentes, **caractérisée en ce que** le polymère gélifiant aqueux associatif est un polyéther polyuréthane choisi parmi :
- le polycondensat de polyéthylèneglycol contenant 136 moles d'oxyde d'éthylène, d'alcool stéarylique polyoxyéthylénisé à 100 moles d'oxyde d'éthylène et d'hexaméthylène diisocyanate ;
- le polycondensat de polyéthylèneglycol à 240 moles d'oxyde d'éthylène, d'alcool décyltétradécylique polyoxyéthylénisé contenant 20 motifs d'oxyde d'éthylène et de diisocyanate d'hexamethylène ;
- un polycondensat de polyéthylèneglycol contenant 150 ou 180 moles d'oxyde d'éthylène, d'alcool stéarylique et de méthylène-bis(4-cyclohexyl-isocyanate) ;
- un polycondensat de polyéthylèneglycol contenant 150 ou 180 moles d'oxyde d'éthylène, d'alcool décylique et de méthylène-bis(4-cyclohexylisocyanate) ;
- un polycondensat de polyéthylèneglycol contenant 200 moles d'oxyde d'éthylène, d'éther de polyéthylèneglycol contenant 10 moles d'oxyde d'éthylène et de méthylglucose, d'éther de polyéthylèneglycol contenant 6 moles d'oxyde d'éthylène et d'alcool tridécylique, de diisocyanate d'hexamethylène, et contenant des groupes terminaux de type alcool en C16-C20 ;
et de préférence choisi parmi :
- le polycondensat de polyéthylèneglycol contenant 136 moles d'oxyde d'éthylène, d'alcool stéarylique polyoxyéthylénisé comportant 100 moles d'oxyde d'éthylène et de diisocyanate d'hexamethylène ;
- le polycondensat de polyéthylèneglycol contenant 240 moles d'oxyde d'éthylène, d'alcool décyltetradécylique polyoxyéthylénisé ayant 20 motifs d'oxyde d'éthylène et de diisocyanate d'hexamethylène ;
- un polycondensat de polyéthylèneglycol contenant 150 ou 180 moles d'oxyde d'éthylène, d'alcool décylique et de méthylène-bis(4-cyclohexylisocyanate) .

22. Composition selon l'une des revendications précédentes, **caractérisée en ce que** le polymère gélifiant hydrophile associatif est présent en une teneur allant de 0,1 % à 2 % en poids, de préférence allant de 0,2 % à 1,5 % en poids, et préférentiellement allant de 0,2 % à 1 % en poids, par rapport au poids total de la composition.

23. Composition selon l'une des revendications précédentes, **caractérisée en ce qu'**elle comprend un gélifiant aqueux supplémentaire choisi parmi :
- les homopolymères d'acide acrylique réticulés, de préférence réticulés avec un agent réticulant choisi parmi l'éther d'allyle de pentaérythritol, l'éther d'allyle de saccharose et l'éther d'allyle de propylène ;
- les carraghénanes, de préférence les iota-carraghénanes ;
- les sels de métal alcalin d'homopolymère d'acide acrylique, de préférence les homopolymères de poly(acrylate de sodium).

24. Composition selon l'une des revendications précédentes, **caractérisée en ce qu'**elle comprend un gélifiant aqueux supplémentaire choisi parmi les homopolymères polyacrylate de sodium.

25. Composition selon l'une ou l'autre des revendications 23 et 24, **caractérisée en ce que** le gélifiant aqueux supplémentaire est présent dans la composition selon l'invention en une teneur allant de 0,1 % à 5 % en poids, de préférence allant de 0,1 % à 3 % en poids, et préférentiellement allant de 0,1 % à 2 % en poids, et mieux allant de 0,2 % à 1,5 % en poids, par rapport au poids total de la composition.

26. Composition selon l'une des revendications précédentes, **caractérisée en ce qu'**elle comprend une huile.

27. Composition selon l'une des revendications précédentes, **caractérisée en ce qu'**elle comprend une huile présente en une teneur allant de 0 % à 30 % en poids, de préférence allant de 0 % à 20 % en poids, préférentiellement allant de 0 % à 15 % en poids et mieux allant de 5 à 15 % en poids, par rapport au poids total de la composition.

28. Composition selon l'une des revendications précédentes, **caractérisée en ce qu'**elle comprend une huile, l'huile et les première et deuxième substances grasses solides étant présentes en un rapport pondéral (première et deuxième substances grasses solides) / huile allant de 0,5 à 1,5, de préférence allant de 0,6 à 1,4, préférentiellement allant de 0,7 à 1,3, et encore mieux allant de 0,9 à 1,2.

29. Composition selon l'une des revendications précédentes, **caractérisée en ce qu'**elle comprend un tensioactif non ionique non de silicone.

30. Composition selon la revendication précédente, **caractérisée en ce que** le tensioactif non ionique non de silicone est choisi parmi :
- les alcools gras en C8-C30 polyoxyéthylénisés contenant notamment de 2 à 100 moles d'oxyde d'éthylène,
- les éthers d'alcool gras en C8-C30 et de sucre, en particulier des (C8-C30)alkyl-(poly)glucosides,
- les éthers de polyéthylèneglycol, contenant notamment de 20 à 120 motifs d'oxyde d'éthylène, et d'un ester d'acide gras en C8-C30 de glucose ou de méthylglucose,
- les esters d'acide gras en C₈-C₃₀ de sorbitane
- les esters d'acides gras en C8-C30 polyoxyéthylénisés de sorbitane, contenant notamment de 2 à 30 moles d'oxyde d'éthylène,
- les esters polyoxyéthylénisés d'acide gras en C8-C30 de sorbitane, contenant notamment de 2 à 100 moles d'oxyde d'éthylène,
- les monoesters ou les diesters d'acide gras en C8-C30 de glycérol,
- les esters d'acides gras en C8-C30 polyglycérolés, contenant notamment de 2 à 16 moles de glycérol,
- les esters d'acide gras en C8-C30 de polyéthylèneglycol, ayant notamment de 2 à 200 motifs d'oxyde d'éthylène,
- les esters d'acide gras en C8-C30 de glucose ou d'alkyl(C1-C2)glucose ou de saccharose,
- et des mélanges de ceux-ci ;
de préférence choisi parmi les alcools gras en C8-C30 polyoxyéthylénisés, notamment contenant de 2 à 100 moles d'oxyde d'éthylène et les alkyl(C8-C30) (poly)glucosides ;
préférentiellement, choisi parmi les alcools gras en C8-C30 polyoxyéthylénisés, notamment contenant de 2 à 100 moles d'oxyde d'éthylène.

31. Composition selon la revendication 29 ou 30, **caractérisée en ce que** le tensioactif non ionique non de silicone est présent dans la composition selon l'invention en une teneur allant de 0,1 % à 4 % en poids, de préférence de 0,1 % à 3 % en poids, et mieux encore de 0,4 % à 2,5 % en poids, et même encore mieux allant de 0,4 % à 2 % en poids, par rapport au poids total de la composition.

32. Composition selon l'une des revendications précédentes, **caractérisée en ce qu'**elle comprend de l'eau en une teneur allant de 25 % à 90 % en poids, de préférence de 30 % à 80 % en poids, et mieux encore de 40 % à 70 % en poids, par rapport au poids total de la composition.

33. Composition selon l'une des revendications précédentes, **caractérisée en ce qu'**elle comprend un organopolysiloxane élastomère non émulsifiant.

34. Composition selon l'une des revendications précédentes, **caractérisée en ce qu'**elle possède une dureté, à 25 °C, allant de 1 à 30 kPa, de préférence allant de 1 à 15 kPa et préférentiellement allant de 1 à 10 kPa.

35. Procédé, notamment procédé cosmétique, pour le traitement de la peau, comprenant l'application à la peau d'une composition selon l'une des revendications précédentes.

36. Procédé selon la revendication précédente, **caractérisé en ce que** le procédé est destiné au traitement du relâchement cutané et/ou à la réduction de l'apparence des ridules de la peau.
